(19) Europäisches Patentamt / European Patent Office / Office européen des brevets

(11) **EP 4 232 515 B1**

(12) **EUROPEAN PATENT SPECIFICATION**

(45) Date of publication and mention
of the grant of the patent:
**29.07.2026  Bulletin 2026/31**

(21) Application number: **21798341.0**

(22) Date of filing: **20.10.2021**

(51) International Patent Classification (IPC):
**C09C 1/02** *(2006.01)*

(52) Cooperative Patent Classification (CPC):
**C09C 1/021; C09C 1/024;** C01P 2002/82;
C01P 2002/84; C01P 2004/84; C01P 2006/12;
C01P 2006/14; C01P 2006/62; C01P 2006/63;
C01P 2006/64

(86) International application number:
**PCT/EP2021/079100**

(87) International publication number:
**WO 2022/084392 (28.04.2022 Gazette 2022/17)**

(54) **METHOD FOR PRODUCING SURFACE-REACTED CALCIUM CARBONATE FUNCTIONALIZED WITH IRON OXIDE SPECIES FOR COSMETIC, PAINT AND COATING APPLICATIONS**

VERFAHREN ZUR HERSTELLUNG VON MIT EISENOXID-SPEZIES FUNKTIONALISIERTES OBERFLÄCHENREAGIERTES CALCIUMCARBONAT FÜR KOSMETIK-, FARB- UND BESCHICHTUNGSANWENDUNGEN

METHODE DE FABBRICATION DE CARBONATE DE CALCIUM TRAITÉ PAR RÉACTION EN SURFACE FONCTIONNALISÉ PAR DES ESPÈCES D'OXYDE DE FER POUR APPLICATIONS COSMÉTIQUES, DE PEINTURE ET DE REVÊTEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2020  EP 20203109**

(43) Date of publication of application:
**30.08.2023  Bulletin 2023/35**

(73) Proprietor: **Omya International AG**
**4665 Oftringen (CH)**

(72) Inventors:
• **FTOUNI, Jamal**
  **4800 Zofingen (CH)**
• **ORLANDO, Fabrizio**
  **5436 Würenlos (CH)**

(74) Representative: **Maiwald GmbH**
**Elisenhof**
**Elisenstraße 3**
**80335 München (DE)**

(56) References cited:
**EP-A1- 3 192 838      EP-A1- 3 360 601**
**WO-A1-2018/019692**

**Description**

The present invention relates to a method for the manufacture of a pigment, the use of the pigment in cosmetic applications, paint and coating applications, as well as products comprising the pigment.

Pigments are coloured material, mostly inorganic compounds, which are completely or nearly insoluble in water.

Like all materials, the colour of pigments arises because they absorb only certain wavelengths of visible light. The bonding properties of the material determine the wavelength and efficiency of light absorption. Light of other wavelengths are reflected or scattered. The reflected light spectrum defines the colour.

Other properties of a colour, such as its saturation or lightness, may be determined by the other substances that accompany pigments. Also binders and fillers can affect the colour.

Minerals have been used as colourants since prehistoric times. For example, Red Ochre, anhydrous $Fe_2O_3$, and the hydrated Yellow Ochre ($Fe_2O_3 H_2O$).

Pigments have advantages in many respects such as lightfastness and sensitivity for damage from ultraviolet light, heat stability, tinting strength, staining, dispersion, control of opacity or transparency, resistance to alkalis and acids, reactions and interactions between pigments, etc.

One group of inorganic pigments are iron oxide pigments. There are sixteen known iron oxides and oxyhydroxides, the best known of which is rust, a form of iron(III) oxide.

Iron oxides and oxyhydroxides are widespread in nature and play an important role in many geological and biological processes, and they are used as pigments, as they are inexpensive and durable, e.g. in paints, coatings and coloured concretes. Colours commonly available are in the "earthy" end of the yellow/orange/red/brown/black range. They may even be used as a food colouring.

In colour cosmetics, iron oxide pigments are used to provide colour. These pigments are widely used because of their low toxicity and large availability. In a formulation, these coloured pigments are mixed physically with the other components of the cosmetic formulation to achieve the suitable colours, such as with titan dioxide, talc etc.. The use of treated surface-reacted calcium carbonate, surface-treated surface-reacted calcium carbonate or surface-reacted calcium carbonate in *inter alia* cosmetics, paints and/or coatings is for example disclosed in EP3192838A1, WO2018019692A1 or EP3360601A1.

However, to achieve certain shades of colour, often a high amount of metallic pigments is required, as different compounds have to be mixed to achieve a certain colour or shade.

Thus, iron oxide pigments are well known, and widely used due to their advantageous properties. Nevertheless, there is the need for improving these pigments, and their use in formulations.

Accordingly, it is the object of the present invention to provide pigments and a method for their manufacture providing a wide range of different colours and shades in an easy, material saving, efficient way, wherein the obtained pigments may be used in a number of applications, inter alia in cosmetic applications, i.e. are not harmful to health, e.g. if applied to the skin, have pleasant sensorial effects, improved optical properties, including their UV absorption properties and IR reflectance, e.g. provide UV protection. Furthermore the pigments should have a good colour consistency, dispersibility, as well as a good safety and sustainability profile.

A further object is the use of such a pigment in cosmetic, paint and coating applications.

Furthermore, products, especially cosmetic, paint and coating products comprising the inventive pigment are an object of the present invention.

It has now surprisingly been found that by decorating surface-reacted calcium carbonate with iron oxide species, which are obtained starting from iron salts, not only allows for a decrease of the used amount of iron but also for obtaining new shades of colours.

Unlike the commercial products, in which the metal species are physically combined/mixed with the calcium carbonate, the inventive product consists in decorating, i.e. coating the surface-reacted calcium carbonate surface with the metal species to result in coated particles instead of a mixture of distinct calcium carbonate and pigment particles. Thus, the iron content may be reduced in the final product by up to 50 %.

This new method and resulting pigments provide new unique colours, excellent optical properties, such as UV protection, and distinctive sensorial effects.

The new inorganic mineral based pigments have a number of further advantages such as outstanding colour consistency, an excellent dispersibility, a great selection of warm colours. They replace some features of mica, silica, or $TiO_2$, which are usual additives in cosmetic, paint and coating applications.

The pigments according to the invention provide an excellent safety and sustainability profile and enable customized solutions.

Accordingly, the foregoing and other objects are solved by the subject-matter as defined in the independent claims. Advantageous embodiments of the present invention are defined in the corresponding subclaims.

It should be understood that, for the purpose of the present invention, the following terms have the following meaning:

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

Accordingly, in a first aspect, the present invention relates to a method for the manufacture of a pigment characterized by the steps of

a) providing at least one surface-reacted calcium carbonate,
b) providing at least one water-soluble iron compound,
c) providing at least one treatment agent,
d) combining the at least one surface-reacted calcium carbonate of step a) with the at least one water-soluble iron compound of step b) in an aqueous medium
e) adding the at least one treatment agent to the mixture of step d),
f) dewatering the mixture of step e),
g) thermally treating the mixture of step f) at a temperature of from 80 to 150 °C,

wherein the surface-reacted calcium carbonate is a reaction product of ground natural calcium carbonate-containing mineral (GNCC) or precipitated calcium carbonate (PCC) with carbon dioxide and one or more $H_3O^+$ ion donors and wherein the carbon dioxide is formed in situ by the $H_3O^+$ ion donors treatment and/or is supplied from an external source, and

wherein the at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, based on the total dry weight of the surface-reacted calcium carbonate.

A "pigment" in the meaning of the present invention is a coloured inorganic material that is completely or nearly insoluble in water.

A "surface-reacted calcium carbonate" (SRCC) according to the present invention is a reaction product of ground natural calcium carbonate (GNCC) or precipitated calcium carbonate (PCC) treated with carbon dioxide and one or more $H_3O^+$ ion donors, wherein the carbon dioxide is formed in situ by the $H_3O^+$ ion donors treatment and/or is supplied from an external source. A $H_3O^+$ ion donor in the context of the present invention is a Brønsted acid and/or an acid salt.

In a preferred embodiment of the invention, the surface-reacted calcium carbonate is obtained by a process comprising the steps of: (a) providing a suspension of natural or precipitated calcium carbonate, (b) adding at least one acid having a $pK_a$ value of 0 or less at 20°C or having a $pK_a$ value from 0 to 2.5 at 20°C to the suspension of step (a), and (c) treating the suspension of step (a) with carbon dioxide before, during or after step (b). According to another embodiment the surface-reacted calcium carbonate is obtained by a process comprising the steps of: (A) providing a natural or precipitated calcium carbonate, (B) providing at least one water-soluble acid, (C) providing gaseous $CO_2$, (D) contacting said natural or precipitated calcium carbonate of step (A) with the at least one acid of step (B) and with the $CO_2$ of step (C), characterised in that: (i) the at least one acid of step B) has a $pK_a$ of greater than 2.5 and less than or equal to 7 at 20°C, associated with the ionisation of its first available hydrogen, and a corresponding anion is formed on loss of this first available hydrogen capable of forming a water-soluble calcium salt, and (ii) following contacting the at least one acid with natural or precipitated calcium carbonate, at least one water-soluble salt, which in the case of a hydrogen-containing salt has a $pK_a$ of greater than 7 at 20°C, associated with the ionisation of the first available hydrogen, and the salt anion of which is capable of forming water-insoluble calcium salts, is additionally provided.

"Natural ground calcium carbonate" (GCC) preferably is selected from calcium carbonate containing minerals selected from the group comprising marble, chalk, limestone and mixtures thereof. Natural calcium carbonate may comprise further naturally occurring components such as alumino silicate etc.

In general, the grinding of natural ground calcium carbonate may be a dry or wet grinding step and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulveriser, a shredder, a de-clumper, a knife cutter, or other such equipment known to the skilled man. In case the calcium carbonate containing mineral material comprises a wet ground calcium carbonate containing mineral material, the grinding step may be performed under conditions such that autogenous grinding takes place and/or by horizontal ball milling, and/or other such processes known to the skilled man. The wet processed ground calcium carbonate containing mineral material thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

"Precipitated calcium carbonate" (PCC) in the meaning of the present invention is a synthesized material, generally obtained by precipitation following reaction of carbon dioxide and calcium hydroxide in an aqueous environment or by precipitation of calcium and carbonate ions, for example $CaCl_2$ and $Na_2CO_3$, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained PCC slurry can be mechanically dewatered and dried.

According to one embodiment of the present invention, the precipitated calcium carbonate is precipitated calcium carbonate, preferably comprising aragonitic, vateritic or calcitic mineralogical crystal forms or mixtures thereof.

Precipitated calcium carbonate may be ground prior to the treatment with carbon dioxide and at least one $H_3O^+$ ion donor by the same means as used for grinding natural calcium carbonate as described above.

According to one embodiment of the present invention, the natural or precipitated calcium carbonate is in form of particles having a weight median particle size $d_{50}$ of 0.05 to 10.0 $\mu$m, preferably 0.2 to 5.0 $\mu$m, more preferably 0.4 to 3.0 $\mu$m, most preferably 0.6 to 1.2 $\mu$m, especially 0.7 $\mu$m. According to a further embodiment of the present invention, the natural or precipitated calcium carbonate is in form of particles having a top cut particle size $d_{98}$ of 0.15 to 55 $\mu$m, preferably 1 to 40 $\mu$m, more preferably 2 to 25 $\mu$m, most preferably 3 to 15 $\mu$m, especially 4 $\mu$m.

The natural and/or precipitated calcium carbonate may be used dry or suspended in water. Preferably, a corresponding slurry has a content of natural or precipitated calcium carbonate within the range of 1 wt% to 90 wt%, more preferably 3 wt% to 60 wt%, even more preferably 5 wt% to 40 wt%, and most preferably 10 wt% to 25 wt% based on the weight of the slurry.

The one or more $H_3O^+$ ion donor used for the preparation of surface-reacted calcium carbonate may be any strong acid, medium-strong acid, or weak acid, or mixtures thereof, generating $H_3O^+$ ions under the preparation conditions. According to the present invention, the at least one $H_3O^+$ ion donor can also be an acidic salt, generating $H_3O^+$ ions under the preparation conditions.

According to one embodiment, the at least one $H_3O^+$ ion donor is a strong acid having a $pK_a$ of 0 or less at 20°C.

According to another embodiment, the at least one $H_3O^+$ ion donor is a medium-strong acid having a $pK_a$ value from 0 to 2.5 at 20°C. If the $pK_a$ at 20°C is 0 or less, the acid is preferably selected from sulphuric acid, hydrochloric acid, or mixtures thereof. If the $pK_a$ at 20°C is from 0 to 2.5, the $H_3O^+$ ion donor is preferably selected from $H_2SO_3$, $H_3PO_4$, oxalic acid, or mixtures thereof. The at least one $H_3O^+$ ion donor can also be an acidic salt, for example, $HSO_4^-$ or $H_2PO_4^-$, being at least partially neutralized by a corresponding cation such as $Li^+$, $Na^+$ or $K^+$, or $HPO_4^{2-}$, being at least partially neutralised by a corresponding cation such as $Li^+$, $Na^+$, $K^+$, $Mg^{2+}$ or $Ca^{2+}$. The at least one $H_3O^+$ ion donor can also be a mixture of one or more acids and one or more acidic salts.

According to still another embodiment, the at least one $H_3O^+$ ion donor is a weak acid having a $pK_a$ value of greater than 2.5 and less than or equal to 7, when measured at 20°C, associated with the ionization of the first available hydrogen, and having a corresponding anion, which is capable of forming water-soluble calcium salts. Subsequently, at least one water-soluble salt, which in the case of a hydrogen-containing salt has a $pK_a$ of greater than 7, when measured at 20°C, associated with the ionization of the first available hydrogen, and the salt anion of which is capable of forming water-insoluble calcium salts, is additionally provided. According to the preferred embodiment, the weak acid has a $pK_a$ value from greater than 2.5 to 5 at 20°C, and more preferably the weak acid is selected from the group consisting of acetic acid, formic acid, propanoic acid, and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of drop wise addition, this addition preferably takes place within a time period of 10 minutes. It is more preferred to add said salt in one step.

According to one embodiment of the present invention, the at least one $H_3O^+$ ion donor is selected from the group consisting of hydrochloric acid, sulphuric acid, sulphurous acid, phosphoric acid, citric acid, oxalic acid, acetic acid, formic acid, and mixtures thereof. Preferably the at least one $H_3O^+$ ion donor is selected from the group consisting of hydrochloric acid, sulphuric acid, sulphurous acid, phosphoric acid, oxalic acid, $H_2PO_4^-$, being at least partially neutralised by a corresponding cation such as $Li^+$, $Na^+$ or $K^+$, $HPO_4^{2-}$, being at least partially neutralised by a corresponding cation such as $Li^+$, $Na^+$, $K^+$, $Mg^{2+}$, or $Ca^{2+}$ and mixtures thereof, more preferably the at least one acid is selected from the group consisting of hydrochloric acid, sulphuric acid, sulphurous acid, phosphoric acid, oxalic acid, or mixtures thereof, and most preferably, the at least one $H_3O^+$ ion donor is phosphoric acid.

The one or more $H_3O^+$ ion donor can be added to the suspension as a concentrated solution or a more diluted solution. Preferably, the molar ratio of the $H_3O^+$ ion donor to the natural or precipitated calcium carbonate is from 0.01 to 4, more preferably from 0.02 to 2, even more preferably 0.05 to 1 and most preferably 0.1 to 0.58.

As an alternative, it is also possible to add the $H_3O^+$ ion donor to the water before the natural or precipitated calcium carbonate is suspended.

In a next step, the natural or precipitated calcium carbonate is treated with carbon dioxide. If a strong acid such as sulphuric acid or hydrochloric acid is used for the $H_3O^+$ ion donor treatment of the natural or precipitated calcium carbonate, the carbon dioxide is automatically formed. Alternatively or additionally, the carbon dioxide can be supplied from an external source.

$H_3O^+$ ion donor treatment and treatment with carbon dioxide can be carried out simultaneously which is the case when a strong or medium-strong acid is used. It is also possible to carry out $H_3O^+$ ion donor treatment first, e.g. with a medium strong acid having a $pK_a$ in the range of 0 to 2.5 at 20°C, wherein carbon dioxide is formed in situ, and thus, the carbon dioxide treatment will automatically be carried out simultaneously with the $H_3O^+$ ion donor treatment, followed by the additional treatment with carbon dioxide supplied from an external source.

In a preferred embodiment, the $H_3O^+$ ion donor treatment step and/or the carbon dioxide treatment step are repeated at least once, more preferably several times. According to one embodiment, the at least one $H_3O^+$ ion donor is added over a time period of at least about 5 min, preferably at least about 10 min, typically from about 10 to about 20 min, more preferably about 30 min, even more preferably about 45 min, and sometimes about 1 h or more.

Subsequent to the $H_3O^+$ ion donor treatment and carbon dioxide treatment, the pH of the aqueous suspension, measured at 20°C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

In a particular preferred embodiment the surface-reacted calcium carbonate is a reaction product of natural ground calcium carbonate (GNCC) with carbon dioxide and phosphoric acid, wherein the carbon dioxide is formed in situ by the phosphoric acid treatment.

Further details about the preparation of the surface-reacted natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A1.

Similarly, surface-reacted precipitated calcium carbonate is obtained. As can be taken in detail from WO 2009/074492 A1, surface-reacted precipitated calcium carbonate is obtained by contacting precipitated calcium carbonate with $H_3O^+$ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by $H_3O^+$ ions, where said $H_3O^+$ ions are provided solely in the form of a counterion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said $H_3O^+$ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural or precipitated calcium carbonate, the natural or precipitated calcium carbonate is reacted with the one or more $H_3O^+$ ion donors and/or the carbon dioxide in the presence of at least one compound selected from the group consisting of silicate, silica, aluminium hydroxide, earth alkali aluminate such as sodium or potassium aluminate, magnesium oxide, or mixtures thereof. Preferably, the at least one silicate is selected from an aluminium silicate, a calcium silicate, or an earth alkali metal silicate. These components can be added to an aqueous suspension comprising the natural or precipitated calcium carbonate before adding the one or more $H_3O^+$ ion donors and/or carbon dioxide.

Alternatively, the silicate and/or silica and/or aluminium hydroxide and/or earth alkali aluminate and/or magnesium oxide component(s) can be added to the aqueous suspension of natural or precipitated calcium carbonate while the reaction of natural or precipitated calcium carbonate with the one or more $H_3O^+$ ion donors and carbon dioxide has already started. Further details about the preparation of the surface-reacted natural or precipitated calcium carbonate in the presence of at least one silicate and/or silica and/or aluminium hydroxide and/or earth alkali aluminate component(s) are disclosed in WO 2004/083316.

The surface-reacted calcium carbonate can be kept in suspension, optionally further stabilised by a dispersant. Conventional dispersants known to the skilled person can be used. A preferred dispersant is comprised of polyacrylic acids and/or carboxymethylcelluloses.

Alternatively, the aqueous suspension described above can be dried, thereby obtaining the solid (i.e. dry or containing as little water that it is not in a fluid form) surface-reacted natural or precipitated calcium carbonate in the form of granules or a powder.

A "dry" material (e.g. dry surface-reacted calcium carbonate) may be defined by its total moisture content which, unless specified otherwise, is less than or equal to 0.5 wt.%, even more preferably less than or equal to 0.2 wt.%, and most preferably between 0.03 and 0.07 wt.%, based on the total weight of the dried material. The "total moisture content" of a material may be measured according to the Karl Fischer coulometric titration method determining the percentage of moisture (e.g. water), which may be desorbed from a sample upon heating to 220 °C.

A "suspension" or "slurry" in the meaning of the present invention refers to a mixture comprising at least one insoluble solid in a liquid medium, for example water, and optionally further additives, and usually contains large amounts of solids and, thus, is more viscous (higher viscosity) and can have a higher density than the liquid medium from which it is formed.

In a preferred embodiment the particles of surface-reacted calcium carbonate of step a) have a volume median particle size $d_{50}$ (vol) of from 1 to 75 $\mu$m, preferably from 2 to 50 $\mu$m, more preferably 3 to 40 $\mu$m, even more preferably from 4 to 30 $\mu$m, and most preferably from 5 to 15 $\mu$m.

It may furthermore be preferred that the particles of surface-reacted calcium carbonate of step a) have a top cut particle size $d_{98}$ (vol) of from 2 to 150 $\mu$m, preferably from 4 to 100 $\mu$m, more preferably 6 to 80 $\mu$m, even more preferably from 8 to 60 $\mu$m, and most preferably from 10 to 30 $\mu$m.

The value $d_x$ represents the diameter relative to which x % of the particles have diameters less than $d_x$. This means that the $d_{98}$ value is the particle size at which 98 % of all particles are smaller. The $d_{98}$ value is also designated as "top cut". The $d_x$ values may be given in volume or weight percent. The $d_{50}$ (wt) value is thus the weight median particle size, i.e. 50 wt% of all particles are smaller than this particle size, and the $d_{50}$ (vol) value is the volume median particle size, i.e. 50 vol% of all particles are smaller than this particle size.

In a further preferred embodiment, the surface-reacted calcium carbonate has a specific surface area (BET) of from 10 m$^2$/g to 200 m$^2$/g, preferably from 20 m$^2$/g to 180 m$^2$/g, more preferably from 30 m$^2$/g to 160 m$^2$/g, even more preferably from 45 m$^2$/g to 150 m$^2$/g, most preferably from 50 m$^2$/g to 100 m$^2$/g, measured using the BET nitrogen method. For example, the surface-reacted calcium carbonate has a specific surface area of from 75 m$^2$/g to 96 m$^2$/g, measured using nitrogen and the BET method.

Preferably, the surface-reacted calcium carbonate has an intra-particle intruded specific pore volume in the range from 0.1 to 2.3 cm$^3$/g, more preferably from 0.2 to 2.0 cm$^3$/g, especially preferably from 0.4 to 1.8 cm$^3$/g and most preferably from 0.6 to 1.6 cm$^3$/g, calculated from mercury porosimetry measurement.

The intra-particle pore size of the surface-reacted calcium carbonate preferably is in a range of from 0.004 to 1.6 $\mu$m, more preferably in a range of from 0.005 to 1.3 $\mu$m, especially preferably from 0.006 to 1.15 $\mu$m and most preferably of 0.007 to 1.0 $\mu$m, e.g. 0.01 to 0.1 $\mu$m determined by mercury porosimetry measurement.

The at least one water-soluble iron compound of step b) may be added in dry form or in the form of an aqueous solution, and, preferably, is selected from inorganic water-soluble iron(II) and/or iron(III) salts. In an especially preferred embodiment the at least one water-soluble iron compound of step b) is selected from the group comprising iron(II) sulfate; iron(III) sulfate; iron(II) halides, such as iron(II) chloride or iron(II) bromide; iron(III) halides, such as iron(III) chloride or iron(III) bromide; iron(II) nitrate; iron(III) nitrate; iron(II) phosphate; iron(III) phosphate; iron(II) oxalate; iron(III) oxalate; iron(II) acetate; iron(III) acetate; hydrates, and mixtures thereof.

A "water-soluble" material in the meaning of the present invention is defined as a material, which, when 100 g of said material is mixed with 100 g deionized water and filtered on a filter having a 0.2 $\mu$m pore size at 20 °C under atmospheric pressure to recover the liquid filtrate, provides more than 0.1 g of recovered solid material following evaporation at 95 to 100 °C of 100 g of said liquid filtrate at ambient pressure. Accordingly, "water-insoluble" materials are defined as materials which, when 100 g of said material are mixed with 100 g deionized water and filtered on a filter having a 0.2 μm pore size at 20 °C under atmospheric pressure to recover the liquid filtrate, provide less than or equal to 0.1 g of recovered solid material following evaporation at 95 to 100 °C of 100 g of said liquid filtrate at ambient pressure.

In a preferred embodiment, the at least one water-soluble iron compound of step b) is added in an amount of from 0.05 to 40 wt%, preferably in an amount of from 0.1 to 30 wt%, more preferably in an amount of from 0.5 to 20 wt%, even more preferably in an amount of from 1 to 10 wt%, most preferably in an amount of from 3 to 7.5 wt%, especially preferably in an amount of from 5 wt% to 6 wt% relating to the iron content, in relation to the total dry weight of the surface-reacted calcium carbonate.

For example, if 200 g of surface-reacted calcium carbonate are used, it may be preferred to add 1 g of $FeSO_4 \cdot 7$ $H_2O$ (278,0 g/mol), i.e. 0.5 wt% in relation to the dry weight of surface-reacted calcium carbonate, which is 0.1 wt% relating to the iron content (55,9 g/mol) of $FeSO_4 \cdot 7 H_2O$ in relation to the dry weight of surface-reacted calcium carbonate.

Furthermore, a treatment agent is provided in step c). This treatment agent may be selected from the group comprising precipitation agents and reducing agents.

Precipitation agents according to the present invention are compounds, which are capable to form water-insoluble iron compounds when when combined with the water-soluble iron compound. The precipitation agents of the present invention are preferably selected from the group comprising alkaline and alkaline earth hydroxides, such as sodium hydroxide, potassium hydroxide, calcium hydroxide; ammonia; and mixtures thereof.

In an especially preferred embodiment according to the present invention, the precipitation agent does not comprise carbonate ions and, especially, is not sodium carbonate.

Reducing agents according to the present invention are elements or compounds which lose (or "donate") an electron to an electron recipient, and thus reduce the oxidation state of the recipient. Common reducing agents include metals, potassium, calcium, barium, sodium and magnesium, and also compounds that contain the H⁻ ion, those being NaH, LiH, $LiAlH_4$ and $CaH_2$.

In the present invention, it is preferred, if the reducing agent, once added into water, will release molecular hydrogen to transform the ionic metal species into reduced ones, especially into elemental iron.

Accordingly, especially preferred reducing agents according to the present invention are reducing agents forming elemental iron when combined with the water-soluble iron compound, and are preferably selected from the group comprising sodium borohydride, lithium borohydride, sodium hydride, lithium aluminium hydride, hydrogen, hydrazine, sodium citrate; and mixtures thereof.

The at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, preferably from 0.1 to 30 wt%, more preferably from 0.5 to 20 wt%, even more preferably from 1 to 10 wt%, most preferably from 3 to 7.5 wt%, especially preferably from 5 wt% to 6 wt% based on the total dry weight of the surface-reacted calcium carbonate.

The at least one treatment agent of step c) is provided in an amount of from 0.05 to 20 wt%, preferably in an amount of from 0.1 to 15 wt%, more preferably in an amount of from 0.5 to 10 wt%, even more preferably in an amount of from 1 to 7.5 wt%, most preferably in an amount of from 2 to 5 wt%, especially preferably in an amount of from 3 wt% to 4 wt% based on the iron content of the at least one water-soluble iron compound.

In a preferred embodiment, the at least one treatment agent of step c) is provided in a molar ratio of treatment agent / Fe of from 1 : 1 to 15 : 1, preferably 1.5 : 1 to 10 : 1, more preferably 2 : 1 to 7 : 1, most preferably 3 : 1 to 5 : 1.

The ratio treatment agent / Fe relates to the molar ratio of treatment agent to iron content of the water-soluble iron compound of step b).

In step d) the at least one surface-reacted calcium carbonate of step a) is combined with the at least one water-soluble iron compound of step b) in an aqueous medium.

Subsequently, in step e), the at least one treatment agent is added to the mixture of step d).

It is preferred that the treatment agent, especially the precipitation agent, is not added before and/or during step d) to the at least one surface-reacted calcium carbonate of step a).

Both of steps d) and/or e), independently from each other, may be carried out under stirring and/or at a temperature of from 25 to 95 °C, preferably of from 30 to 75 °C, more preferably of from 40 to 65°C, most preferably at 50 °C.

Stirring may be carried out by any equipment suitable therefor, e.g. by a magnetic stirrer or a high speed mixer.

Subsequently, the resulting mixture is dewatered in step f), as, according to the present invention, it is not advantageous to heat the mixture resulting from step e) in the presence of too much water. Therefore, the mixture is dewatered before the thermal treatment step.

Dewatering in the meaning of the present invention means reducing the water content to a level of above 0.5 wt%, but less than 20 wt%, preferably 5 to 15 wt%, e.g. 10 wt%.

Dewatering step f) may be carried out mechanically, thermally, or by a combination of first mechanical and then thermal dewatering, optionally under vacuum.

Mechanical dewatering may be carried out in one or more of a centrifuge, a filtration device, a rotary vacuum filter, a filter press and/or tube press.

Thermal dewatering may be carried out by one or more of a spray dryer and a heat exchanger, jet dryer, oven, compartment dryer, vacuum dryer, microwave dryer and/or freeze dryer.

Subsequently, the dewatered reaction mixture is thermally treated in step g) to obtain a pigment according to the invention. The thermal treatment step may be carried out by conventional methods, such as in an oven, in a special embodiment under vacuum or static air, by jet or spray drying. Thermal treatment step g) is carried out at a temperature of from 80 to 150 °C, preferably of from 90 to 140 °C, more preferably 100 to 130 °C, even more preferably 110 to 125 °C.

In an especially preferred embodiment, after thermal treatment step g), a further thermal treatment step h) is carried out at a temperature of from more than 150 to 600 °C, preferably of from 200 °C to 550 °C, more preferably of from 250 to 500 °C, most preferably of from 300 °C to 450 °C, especially preferably from 350 to 400 °C.

Thermal treatment step h) preferably is a calcination step. "Calcining" or "calcination" in the meaning of the present invention refers to a thermal treatment process applied to solid materials causing loss of moisture, reduction or oxidation, and the decomposition of compounds resulting in an oxide or oxyhydroxide of the corresponding solid material. According to the present invention, the water-insoluble iron compound or elemental iron, which is formed on the surface of the surface-reacted calcium carbonate is transformed into iron oxides, and/or oxyhydroxides, e.g. $FeO(OH)$, $\gamma$-$Fe_2O_3$ and $\alpha$-$Fe_2O_3$.

Calcining may be carried out in any equipment suitable therefor, e.g. in a muffle oven under static air.

Accordingly, in an especially preferred embodiment, thermal treatment step h) is carried out at a temperature of from more than 250 °C to 600 °C, preferably from 300 °C to 500 °C, e.g. 400 °C.

Thermal treatment steps g) and h) may be carried out in two steps, or in one step.

In an especially preferred embodiment, the method according to the invention as defined above is characterized by the steps of

    a) providing at least one surface-reacted calcium carbonate,
    b) providing at least one water-soluble iron compound,
    c) providing at least one precipitation agent, which preferably does not comprise carbonate ions and more preferably is not sodium carbonate,
    d) combining the at least one surface-reacted calcium carbonate of step a) with the at least one water-soluble iron compound of step b) in an aqueous medium,
    e) adding the at least one precipitation agent to the mixture of step d),
    f) dewatering the mixture of step e),
    g) thermally treating the mixture of step f) at a temperature of from 80 to 150 °C, and, optionally
    h) thermally treating the mixture of step g) at a temperature of from more than 150 to 600 °C,

    wherein the surface-reacted calcium carbonate is a reaction product of ground natural calcium carbonate-containing mineral (GNCC) or precipitated calcium carbonate (PCC) with carbon dioxide and one or more $H_3O^+$ ion donors and wherein the carbon dioxide is formed in situ by the $H_3O^+$ ion donors treatment and/or is supplied from an external source, and
    wherein the at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, based on the total dry weight of the surface-reacted calcium carbonate.

In a further especially preferred embodiment, the method according to the invention as defined above is characterized by the steps of

    a) providing at least one surface-reacted calcium carbonate,
    b) providing at least one water-soluble iron compound,
    c) providing at least one reducing agent,
    d) combining the at least one surface-reacted calcium carbonate of step a) with the at least one water-soluble iron compound of step b) in an aqueous medium,
    e) adding the at least one reducing agent to the mixture of step d),
    f) dewatering the mixture of step e),
    g) thermally treating the mixture of step f) at a temperature of from 80 to 150 °C, and, optionally
    h) thermally treating the mixture of step g) at a temperature of from more than 150 to 600 °C,

    wherein the surface-reacted calcium carbonate is a reaction product of ground natural calcium carbonate-containing mineral (GNCC) or precipitated calcium carbonate (PCC) with carbon dioxide and one or more $H_3O^+$ ion donors and

wherein the carbon dioxide is formed in situ by the $H_3O^+$ ion donors treatment and/or is supplied from an external source, and

wherein the at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, based on the total dry weight of the surface-reacted calcium carbonate.

Thus by varying the amount of iron compound in relation to the surface-reacted calcium carbonate, by varying the molar ratio of treatment agent to iron, and by varying the temperature of the thermal treatment step, a number of new pigments can be obtained having different colours and colour shades as very illustratively shown in the Examples.

These new pigments have excellent reflectance and absorption properties in the UV and visible range, and are well tolerated on the skin, such that they may not only advantageously be used in conventional paint and coating applications, but also especially in cosmetic applications. It may also be used in cool painting formulations due to its IR reflective properties.

It has also turned out that less pigment has to be used compared with mixtures of surface-reacted calcium carbonate with known iron oxide based pigments.

Also disclosed herein is a pigment obtained by the above described method of the invention.

Furthermore, another aspect of the present invention is the use of the pigment obtained by the method according to the present invention in cosmetic applications, paint and coating applications.

Finally, a further aspect of the present invention is a product comprising a pigment obtained by the inventive method, which preferably is selected from the group comprising cosmetic products, e.g. eye and face makeup, lipsticks, skin care, toothpaste, or sun protection, and in paints and coatings.

A still further aspect of the present invention is a method for the manufacture of a cosmetic product, paint or coating characterized by the steps of

a) providing at least one surface-reacted calcium carbonate,
b) providing at least one water-soluble iron compound,
c) providing at least one treatment agent,
d) combining the at least one surface-reacted calcium carbonate of step a) with the at least one water-soluble iron compound of step b) in an aqueous medium,
e) adding the at least one treatment agent to the mixture of step d),
f) dewatering the mixture of step e),
g) thermally treating the mixture of step f) at a temperature of from 80 to 150 °C to obtain a pigment,
i) adding the pigment obtained in step g) to a cosmetic formulation, paint or coating,

wherein the surface-reacted calcium carbonate is a reaction product of ground natural calcium carbonate-containing mineral (GNCC) or precipitated calcium carbonate (PCC) with carbon dioxide and one or more $H_3O^+$ ion donors and wherein the carbon dioxide is formed in situ by the $H_3O^+$ ion donors treatment and/or is supplied from an external source, and

wherein the at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, based on the total dry weight of the surface-reacted calcium carbonate.

The preferred embodiments of the method for the manufacture of a pigment as described above also apply to the method for the manufacture of a cosmetic product, paint or coating.

Cosmetic products may be any kind of cosmetic formulations such e.g. those selected from the group comprising creams, lotions, ointments, gels, pastes, aerosol foams or sprays, powders, solids and solutions.

The following figures, examples and tests will illustrate the present invention, but are not intended to limit the invention in any way.

**Figures**

Figure 1 illustrates colours and colour shades obtainable by inventive pigments manufactured using NaOH as the treatment agent

Figure 2 illustrates colours and colour shades obtainable by inventive pigments manufactured using $NaBH_4$ as the

treatment agent

Figure 3 illustrates the reflectance properties of inventive samples having a molar ratio of NaOH : Fe of 1 : 1 and being heat treated at a temperature of 125 °C, and a comparative untreated sample.

Figure 4 illustrates the absorption properties (calculated) of samples having a molar ratio of NaOH : Fe of 1 : 1 and being heat treated at a temperature of 125 °C, and a comparative untreated sample.

Figure 5 illustrates the reflectance properties of samples having a molar ratio of NaOH : Fe of 1 : 1 and being calcined at a temperature of 500 °C, and a comparative untreated sample.

Figure 6 illustrates the absorption properties (calculated) of samples having a molar ratio of NaOH : Fe of 1 : 1 and being calcined at a temperature of 500 °C, and a comparative untreated sample.

Figure 7 illustrates the reflectance properties of samples having a molar ratio of NaOH : Fe of 5 : 1 and being heat treated at a temperature of 125 °C, and a comparative untreated sample.

Figure 8 illustrates the absorption properties (calculated) of samples having a molar ratio of NaOH : Fe of 5 : 1 and being heat treated at a temperature of 125 °C, and a comparative untreated sample.

Figure 9 illustrates the reflectance properties of samples having a molar ratio of NaOH : Fe of 5 : 1 and being calcined at a temperature of 500 °C, and a comparative untreated sample.

Figure 10 illustrates the absorption properties (calculated) of samples having a molar ratio of NaOH : Fe of 5 : 1 and being calcined at a temperature of 500 °C, and a comparative untreated sample.

Figure 11 illustrates the reflectance properties of samples having a molar ratio of $NaBH_4$ : Fe of 1 : 1 and being heat treated at a temperature of 125 °C, and a comparative untreated sample.

Figure 12 illustrates the absorption properties (calculated) of samples having a molar ratio of $NaBH_4$ : Fe of 1 : 1 and being heat treated at a temperature of 125 °C, and a comparative untreated sample.

Figure 13 illustrates the reflectance properties of samples having a molar ratio of $NaBH_4$ : Fe of 1 : 1 and being calcined at a temperature of 500 °C, and a comparative untreated sample.

Figure 14 illustrates the absorption properties (calculated) of samples having a molar ratio of $NaBH_4$ : Fe of 1 : 1 and being calcined at a temperature of 500 °C, and a comparative untreated sample.

Figure 15 illustrates the reflectance properties of samples having a molar ratio of $NaBH_4$ : Fe of 5 : 1 and being heat treated at a temperature of 125 °C, and a comparative untreated sample.

Figure 16 illustrates the absorption properties (calculated) of samples having a molar ratio of $NaBH_4$ : Fe of 5 : 1 and being heat treated at a temperature of 125 °C, and a comparative untreated sample.

Figure 17 illustrates the reflectance properties of samples having a molar ratio of $NaBH_4$ : Fe of 5 : 1 and being calcined at a temperature of 500 °C, and a comparative untreated sample.

Figure 18 illustrates the absorption properties (calculated) of samples having a molar ratio of $NaBH_4$ : Fe of 5 : 1 and being calcined at a temperature of 500 °C, and a comparative untreated sample.

Figure 19 illustrates the results of a comparison of mixtures of surface-reacted calcium carbonate with commercial pigments and inventive pigments.

Figures 20 to 24 illustrate W/O and O/W creams containing the pigments of the invention in different colour ranges.

Figure 25 illustrates the coverage of two formulations comprising pigments according to the invention at a concentration of 5 wt% and 10 wt%.

## EXAMPLES

### 1. Analytical Methods

### Particle size distribution

Volume determined median particle size $d_{50}$(vol) and the volume determined top cut particle size $d_{98}$(vol) as well as the volume particle sizes $d_{90}$(vol) and $d_{10}$(vol) may be evaluated in a wet unit using a Malvern Mastersizer 2000 or 3000 Laser Diffraction System (Malvern Instruments Plc., Great Britain). If not otherwise indicated in the following example section, the volume particle sizes were evaluated in a wet unit using a Malvern Mastersizer 2000 Laser Diffraction System (Malvern Instruments Plc., Great Britain). The $d_{50}$(vol) or $d_{98}$(vol) value indicates a diameter value such that 50 % or 98 % by volume, respectively, of the particles have a diameter of less than this value. The raw data obtained by the measurement was analyzed using the Mie theory, with a particle refractive index of 1.57 and an absorption index of 0.005. The methods and instruments are known to the skilled person and are commonly used to determine particle size distributions of fillers and pigments. The sample was measured in dry condition without any prior treatment.

The weight determined median particle size $d_{50}$(wt) was measured by the sedimentation method, which is an analysis of sedimentation behaviour in a gravimetric field. The measurement was made with a Sedigraph™ 5120 of Micromeritics Instrument Corporation, USA. The method and the instrument are known to the skilled person and are commonly used to determine particle size distributions of fillers and pigments. The measurement was carried out in an aqueous solution of 0.1 wt% $Na_4P_2O_7$. The samples were dispersed using a high speed stirrer and supersonicated.

The processes and instruments are known to the skilled person and are commonly used to determine particle sizes of fillers and pigments.

## BET specific surface area of a material

The "specific surface area" (expressed in $m^2/g$) of a material as used throughout the present document is determined by the Brunauer Emmett Teller (BET) method with nitrogen as adsorbing gas and by use of a ASAP 2460 instrument from Micromeritics. The method is well known to the skilled person and defined in ISO 9277:2010. Samples are conditioned at 100 °C under vacuum for a period of 60 min prior to measurement. The total surface area (in $m^2$) of said material can be obtained by multiplication of the specific surface area (in $m^2/g$) and the mass (in g) of the material.

## Pore Volume / Porosity

For the purpose of the present invention the "porosity" or "pore volume" refers to the intra-particle intruded specific pore volume.

In the context of the present invention, the term "pore" is to be understood as describing the space that is found between and/or within particles, i.e. that is formed by the particles as they pack together under nearest neighbour contact (interparticle pores), such as in a powder or a compact, and/or the void space within porous particles (intraparticle pores), and that allows the passage of liquids under pressure when saturated by the liquid and/or supports absorption of surface wetting liquids.

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 $\mu$m. The equilibration time used at each pressure step is 20 s. The sample material is sealed in a 5 $cm^3$ chamber powder penetrometer for analysis. The data are corrected for mercury compression, penetrometer expansion and sample material elastic compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 1996, 35(5), 1753 - 1764).

The total pore volume seen in the cumulative intrusion data is separated into two regions with the intrusion data from 214 $\mu$m down to about 1 to 4 $\mu$m showing the coarse packing of the sample between any agglomerate structures contributing strongly. Below these diameters lies the fine interparticle packing of the particles themselves. If they also have intra particle pores, then this region appears bimodal, and by taking the specific pore volume intruded by mercury into pores finer than the modal turning point, i.e. finer than the bimodal point of inflection, the specific intraparticle pore volume is defined. The sum of these three regions gives the total overall pore volume of the powder, but depends strongly on the original sample compaction/settling of the powder at the coarse pore end of the distribution.

By taking the first derivative of the cumulative intrusion curve, the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

## X-ray diffraction (XRD)

XRD experiments are performed on the samples using rotatable PMMA holder rings. Samples are analyzed with a Bruker D8 Advance powder diffractometer obeying Bragg's law. This diffractometer comprises a 2.2 kW X-ray tube, a sample holder, a $\theta$-$\theta$-goniometer, and a VÅNTEC-1 detector. Nickel-filtered Cu-K$\alpha$ radiation is employed in all experiments. The profiles are chart recorded automatically using a scan speed of 0.7° per min in 2 $\theta$ (XRD GV_7600). The resulting powder diffraction patterns are classified by mineral content using the DIFFRACsuite software packages EVA and SEARCH, based on reference patterns of the ICDD PDF-2 database (XRD LTM_7603).

Quantitative analysis of diffraction data refers to the determination of amounts of different phases in a multi-phase sample and has been performed using the DIFFRACsuite software package TOPAS. In detail, quantitative analysis allows to determine structural characteristics and phase proportions with quantifiable numerical precision from the experimental data itself. This involves modelling the full diffraction pattern using the Rietveld approach such that the calculated pattern(s) duplicates the experimental one.

## Reflectance and Absorption Analysis

Reflectance analysis is carried out with a double beam PerkinElmer Lambda 950 UV/Vis/NIR spectrophotometer equipped with a 150 mm integrating sphere with PMT and InGaAs detectors. The samples are measured by diffuse reflectance spectroscopy. The analysis is performed with the samples loaded into a sealed aluminum cup for powder samples which is placed flush with the reflectance port of the integrating sphere. Measurements are performed in a specular-excluded configuration, that is, the specular component of the reflected light is lost from the measurement by opening the corresponding section of the integrating sphere.

The spectrophotometer is scanned in the range 250 nm - 2500 nm in steps of 5 nm. A Spectralon white standard is used as 100% baseline. CIE colour coordinates L*, a*, b* are calculated with the OptLab-SPX software using the measured reflectance spectra in the range 380-780 nm, and considering a D65 standardized illuminant with an observer angle of 10 degrees. Given a reference colour ($L_1$,$a_1$,$b_1$) and another colour ($L_2$,$a_2$,$b_2$), their colour difference is evaluated as $\Delta E_{1,2} = \sqrt{(L_2 - L_1)^2 + (a_2 - a_1)^2 + (b_2 - b_1)^2}$ .

To get a proxy for the absorption spectrum of the samples, the measured reflectance spectrum is converted using the Kubelka-Munk equation K-M = K/S = $(1-R)^2/2R$, where R is the reflectance as measured above and K and S are the absorption and scattering coefficient, respectively.

## Covering

The covering power, i.e. the power of the covering agent to cover and/or to opacify the skin surface, can be measured by spreading a cosmetic and/or skin care compositions comprising the covering agent on a contrast paper and subsequently measuring the colour values Rx, Ry, Rz of the composition by the means of a colorimeter. By comparison of the colour values of the cosmetic composition and that of the contrast paper, the contrast is calculated. The contrast directly refers to the covering power. Contrast ratio values are determined according to ISO 2814 at a spreading rate of approx. 20 $m^2/l$. The contrast ratio is calculated as described by the equation below:

$$\text{Contrast ratio [\%]} = \frac{Ry_{\text{black}}}{Ry_{\text{white}}} \times 100\ \%$$

with $Ry_{\text{black}}$ and $Ry_{\text{white}}$ being obtained by the measurement of the colour values.

## 2. Material

### Mineral Material

Surface-reacted calcium carbonate (SRCC) having a $d_{50}$(vol) of 4.5 $\mu$m, a $d_{98}$(vol) of 8.6 $\mu$m; a BET specific surface area of 96 $m^2/g$ with an intra-particle intruded specific pore volume of 1.588 $cm^3/g$ (for the pore diameter range of 0.004 to 0.4 $\mu$m).
It was prepared as follows:
In a mixing vessel, 10 liters of an aqueous suspension of ground limestone calcium carbonate was prepared by adjusting the solids of a ground limestone calcium carbonate having a particle size distribution of 90 wt% below 2 $\mu$m based on the total weight of the ground calcium carbonate, such that a solids content of 15 wt% based on the total weight of the aqueous suspension is obtained.

Whilst mixing the slurry, 2.8 kg phosphoric acid was added in the form of an aqueous solution containing 30 wt% phosphoric acid to said suspension over a period of 10 minutes. Throughout the whole procedure the temperature of the suspension was maintained at 70 °C. After the addition of the acid, the suspension was stirred for additional 5 minutes before removing it from the vessel and drying.

### Water-soluble iron salt

$FeSO_4 \cdot 7\ H_2O$ (from Sigma Aldrich; CAS No. 7782-63-0)

### Treatment agent

NaOH (from Sigma Aldrich; CAS No. 1310-73-2)

NaBH$_4$ (from Sigma Aldrich; CAS No. 16940-66-2)

Commercially available Iron Pigments

Ferroxide® Black 78P (CAS No. 1317-61-9)

Pur Oxy Yellow BC (CAS No. 51274-00-1)

Ferroxide® Red 226P (CAS No. 1309-37-1)

**Solvent**

Distilled water

**3. Pigment Preparation**

**3.1. Preparation using a precipitation agent**

Prior to the preparation, surface-reacted calcium carbonate was dried overnight at 125°C. Subsequently, water was added into the reactor, a 3-neck round bottom flask, followed by the surface-reacted calcium carbonate. The slurry was stirred thoroughly during 30 minutes at room temperature. An aqueous solution of iron(II) sulfate heptahydrate was prepared, by solubilizing it in water. Subsequently, the solution was added dropwise to the surface-reacted calcium carbonate slurry. The mixture was kept under thorough mixing for 60 minutes.

A sodium hydroxide solution was prepared by adding the required amount to water. Then, the solution was added to the slurry and kept under mixing for 60 minutes. The mixture was dewatered via a filtration procedure using Whatman paper filters grade 589, washed with water (50% of the total water volume used during the preparation), then finally dried overnight at 125°C in an oven.

Finally, the powder was manually deagglomerated and calcined in the temperature range of 300°C up to 500°C, for a duration of 2 to 3 hours in a Nabertherm Le 6/11 muffle oven.

As can be taken from table 1, 14 samples were prepared using different amounts of iron (Fe) in the form of a water-soluble iron salt, and sodium hydroxide. The molar ratio of sodium hydroxide to iron (NaOH / Fe) was chosen to be 1 or 5. Each one of the 14 samples, was thermally treated at 125°C (drying), and subsequently at 300°C and 500°C. Thus, in total 52 samples were obtained.

The precipitation using NaOH occurs following the reaction:

$$FeSO_4 + 2\ NaOH \rightarrow Fe(OH)_2 + Na_2SO_4$$

During the dewatering process, the sodium sulfate is removed with the filtrate. Iron hydroxide species are generated on the surface of the surface-reacted calcium carbonate and within its pores.

Table 1

| Sample | SRCC slurry | | Iron sulfate solution | | Sodium hydroxide solution | | wt% Iron based on the amount of SRCC | NaOH / Fe Molar ratio |
|---|---|---|---|---|---|---|---|---|
| | SRCC [g] | Water [ml] | FeSO$_4$ · 7 H$_2$O [g] | Water [ml] | NaOH [g] | Water [ml] | | |
| A | | | 1 | 10 | 0.14 | 1 | 0.1 | 1 |
| B | | | 1 | 10 | 0.72 | 3.6 | 0.1 | 5 |
| C | | | 5 | 50 | 0.7 | 3.5 | 0.5 | 1 |
| D | | | 5 | 50 | 3.5 | 17.5 | 0.5 | 5 |
| E | | | 10 | 100 | 1.4 | 7 | 1 | 1 |
| F | | | 10 | 100 | 7.17 | 35.85 | 1 | 5 |
| G | 200 | 1000 | 30 | 300 | 4.3 | 21.5 | 3 | 1 |
| H | | | 30 | 300 | 21.5 | 107.5 | 3 | 5 |

(continued)

| Sample | SRCC slurry | | Iron sulfate solution | | Sodium hydroxide solution | | wt% Iron based on the amount of SRCC | NaOH / Fe Molar ratio |
| --- | --- | --- | --- | --- | --- | --- | --- | --- |
| | SRCC [g] | Water [ml] | $FeSO_4 \cdot 7 H_2O$ [g] | Water [ml] | NaOH [g] | Water [ml] | | |
| I | | | 50 | 500 | 7.2 | 36 | 5 | 1 |
| J | | | 50 | 500 | 35.8 | 179 | 5 | 5 |
| K | | | 75 | 750 | 10.8 | 54 | 7.5 | 1 |
| L | | | 75 | 750 | 53.7 | 268.5 | 7.5 | 5 |
| M | | | 100 | 1000 | 14 | 70 | 10 | 1 |
| N | | | 100 | 1000 | 71.7 | 358.5 | 10 | 5 |

### 3.2. Preparation using a reducing agent

Prior to the preparation, surface-reacted calcium carbonate was dried overnight at 125°C. Subsequently, water was added into the reactor, a 3-neck round bottom flask, followed by the surface-reacted calcium carbonate. The slurry was stirred thoroughly during 30 minutes at room temperature. An aqueous solution of iron(II) sulfate heptahydrate was prepared, by solubilizing it in water. Subsequently, the solution was added dropwise to the surface-reacted calcium carbonate slurry. The mixture was kept under thorough mixing for 60 minutes.

A $NaBH_4$ solution was prepared by adding the required amount to water. Then, the solution was added to the slurry and kept under mixing for 60 minutes. The mixture was dewatered via a filtration procedure using Whatman paper filters grade 589, washed with water (50% of the total water volume used during the preparation), then finally dried overnight at 125°C in a vacuum oven to prevent the oxidation of the iron species under an oxygen rich atmosphere.

Finally, the powder was deagglomerated and calcined, in the temperature range of 300°C up to 500°C, for a duration of 2 to 3 hours in a Nabertherm Le 6/11 muffle oven.

As can be taken from table 2, 12 samples were prepared using different amounts of iron (Fe) in the form of a water-soluble iron salt and sodium borohydride. The molar ratio of sodium borohydride to iron ($NaBH_4$ / Fe) was chosen to be 1, 5 or 10. Each one of the 12 samples, was thermally treated at 125°C (drying), and subsequently at 300°C and 500°C. Thus, in total 36 samples were obtained.

The reduction using $NaBH_4$ occurs according to the following reaction:

$$NaBH_4 + 2 H_2O \rightarrow NaBO_2 + 4 H_2$$

The formed hydrogen reduces the Fe(II) species to elemental Fe(0). The sodium metaborate is removed during the dewatering process with the filtrate. The elementary iron species are generated on the surface of the surface-reacted calcium carbonate and within its pores.

Table 2

| Sample | SRCC slurry | | $FeSO_4$ solution | | $NaBH_4$ solution | | wt% Fe based on the amount of SRCC | $NaBH_4$ / Fe Molar ratio |
| --- | --- | --- | --- | --- | --- | --- | --- | --- |
| | SRCC [g] | Water [ml] | $FeSO_4 \cdot 7 H_2O$ [g] | Water [ml] | $NaBH_4$ [g] | Water [ml] | | |
| AA | | | 1 | 10 | 0.14 | 1 | 0.1 | 1 |
| BB | | | 1 | 10 | 0.72 | 3.6 | 0.1 | 5 |
| CC | | | 10 | 100 | 1.4 | 7 | 1 | 1 |
| DD | | | 10 | 100 | 7.2 | 35.85 | 1 | 5 |
| EE | | | 30 | 300 | 4.3 | 21.5 | 3 | 1 |
| FF | | | 30 | 300 | 21.5 | 107.5 | 3 | 5 |
| GG | 200 | 1000 | 50 | 500 | 7.2 | 36 | 5 | 1 |
| HH | | | 50 | 500 | 35.8 | 179 | 5 | 5 |

(continued)

| Sample | SRCC slurry | | FeSO₄ solution | | NaBH₄ solution | | wt% Fe based on the amount of SRCC | NaBH₄/ Fe Molar ratio |
|---|---|---|---|---|---|---|---|---|
| | SRCC [g] | Water [ml] | FeSO₄ · 7 H₂O [g] | Water [ml] | NaBH₄ [g] | Water [ml] | | |
| II | | | 100 | 1000 | 14 | 70 | 10 | 1 |
| JJ | | | 100 | 1000 | 71.7 | 358.5 | 10 | 5 |
| CC/10 eq. | | | 1 | 10 | 1.4 | 10 | 1 | 10 |
| FF/10 eq. | | | 30 | 300 | 43 | 210.5 | 3 | 10 |

### 3.3. Preparation without using a treatment agent (comparative examples)

Prior to the preparation, 100 g of surface-reacted calcium carbonate was dried overnight at 125°C. Subsequently, water was added into the reactor, a 3-neck round bottom flask, followed by the surface-reacted calcium carbonate. The slurry was stirred thoroughly during 30 minutes at room temperature. An aqueous solution of 100 g iron(II) sulfate heptahydrate was prepared, by solubilizing it in 1000 ml of water. Subsequently, the solution was added dropwise to the surface-reacted calcium carbonate slurry. The mixture was kept under thorough mixing for 120 minutes.

The mixture was dewatered via a filtration procedure using Whatman paper filters grade 589, washed with water (50% of the total water volume used during the preparation), then finally dried overnight at 125°C in a vacuum oven to prevent the oxidation of the iron species under an oxygen rich atmosphere.

Finally, the powder was deagglomerated and calcined, in the temperature range of 300°C up to 500°C, for a duration of 2 to 3 hours in a Nabertherm Le 6/11 muffle oven.

### 4. Characterization

### 4.1. XRD characterization of the samples

The data presented below are extracted from the XRD analysis. From tables 3 and 4, it can clearly be taken that by varying the iron species, the amount of iron species based on the dry weight of surface-reacted calcium carbonate, the molar ratio of precipitation agent or reducing agents to iron, and the calcination temperature, the species formed on the surface of the surface-reacted calcium carbonate is controllably modified leading to tailor made pigments as regards, colours, colour shades, UV and IR reflectance, as shown below.

The transformation of the water-soluble iron compound into a water-insoluble component or elementary iron deposited on the surface of the surface-treated calcium carbonate leads to a different composition of the species after calcination. Thus, the comparative samples, which were prepared without using a treatment agent, comprise calcium sulfate, whereas the inventive samples do not contain calcium sulfate, i.e. are different in this respect. This is especially important in cosmetic applications in view of the fact that calcium sulfate may cause irritations to the skin, eyes, mucous membranes and the upper respiratory system.

Table 3

| Amount of Fe (wt%) | NaOH/Fe molar ratio | Thermal treatment (°C) | | FeO(OH) | $\gamma Fe_2O_3$ | $\alpha Fe_2O_3$ | $Fe^0$ | $CaSO_4.H_2O$ | $CaSO_4$ |
|---|---|---|---|---|---|---|---|---|---|
| | | 125 | 500 | | | | | | |
| 3 | | X | - | X | X | X[a] | X[a] | - | - |
| 3 | | | X | - | X | X | X[a] | - | - |
| 7.5 | 5 | X | | X | X | X | - | - | - |
| 7.5 | | | X | - | X | X | X[a] | - | - |
| 10 | | X | - | X | X | X[a] | X[a] | - | - |
| 10 | | | X | - | X | X | - | - | - |

(continued)

| Amount of Fe (wt%) | NaOH/Fe molar ratio | Thermal treatment (°C) | | FeO(OH) | $\gamma Fe_2O_3$ | $\alpha Fe_2O_3$ | $Fe^0$ | $CaSO_4.H_2O$ | $CaSO_4$ |
|---|---|---|---|---|---|---|---|---|---|
| | | 125 | 500 | | | | | | |
| 10 | $\infty$[b] (comparative) | X | | - | - | X[a] | X[a] | X | - |
| 10 | | | X | - | - | X[a] | X[a] | - | X |
| [a] Traces; [b] No treatment agent. | | | | | | | | | |

Table 4

| Amount of Fe (wt%) | $NaBH_4$/Fe molar ratio | | Thermal treatment (°C) | | Fe species seen using XRD technique | | | |
|---|---|---|---|---|---|---|---|---|
| | 5 | 10 | 125 | 500 | FeO(OH) | $\gamma Fe_2O_3$ | $\alpha Fe_2O_3$ | $Fe^0$ |
| 3 | X | | X | | - | X[a] | X[a] | X |
| 3 | | X | X | | - | - | - | X |
| 3 | | X | | X | - | X | X | X |
| 5 | X | | X | | - | X[a] | - | X |
| 5 | X | | | X | - | X | X | X |
| 10 | X | | X | | - | - | - | X |
| 10 | X | | | X | X[a] | X | X | X |
| [a] Traces | | | | | | | | |

## 4.2. Colours and Colour Shades

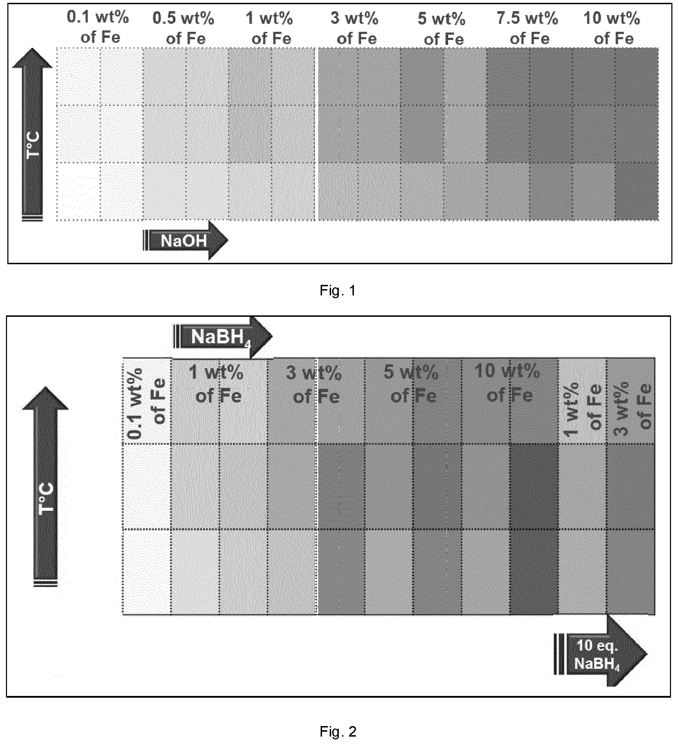

Due to the formation of different Fe species on the surface of the surface-reacted calcium carbonate, it is possible to tailor make pigments of different colours and colour shades from e.g. light beige to dark brown and black, not only by the iron species, the amount of iron species based on the dry weight of surface-reacted calcium carbonate, and the molar ratio of precipitation agent or reducing agents to iron, but also by the calcination temperature, as can be taken from Figures 1 (precipitation agent) and 2 (reducing agent) and tables 5 and 6 reflecting the different shades given in the figures in terms of CIE L*a*b* values. The color analysis was performed on the powdered samples placed in a sample cup as described above.

In figure 1 and table 5, the effects of increasing NaOH/Fe molar ratios (1 : 1 , 5 : 1) at increasing temperatures (125 °C, 300 °C, 500 °C) and increasing iron amounts (0.1 wt%, 0.5 wt%, 1 wt%, 3 wt%, 5 wt%, 7.5 wt%, 10 wt%) are given. In figure 1, the fields from left to right correspond to the composition of samples A to N given in table 5 at the respective temperatures.

In figure 2 and table 6, the effects of increasing $NaBH_4$/Fe molar ratios (1 : 1 , 5 : 1, 10 : 1) at increasing temperatures (125 °C, 300 °C, 500 °C) and increasing iron(0) amounts (0.1 wt%, 1 wt%, 3 wt%, 5 wt%, 10 wt%) are given. In figure 2, the fields from left to right correspond to the composition of samples BB to JJ, and CC with 10 eq. $NaBH_4$, and FF with 10 eq. $NaBH_4$ given in table 6 at the respective temperatures.

Furthermore, $\Delta E$ values are given in tables 5 and 6, which refer to the mixtures of surface-reacted calcium carbonate and commercial pigments mentioned in table 7.

Table 5

| Sample | Temp. [°C] | Elemental Fe [wt%] | NaOH/Fe molar ratio | L* | a* | b* | $\Delta E$ black | $\Delta E$ yellow | $\Delta E$ red |
|---|---|---|---|---|---|---|---|---|---|
| A | 125 | 0.1 | 1 | 97.4 | 0.5 | 3.0 | 50.7 | 48.9 | 46.7 |
| A | 300 | 0.1 | 1 | 96.2 | 1.4 | 4.0 | 49.6 | 47.4 | 45.3 |
| A | 500 | 0.1 | 1 | 95.4 | 1.3 | 3.8 | 48.7 | 47.3 | 44.6 |
| B | 125 | 0.1 | 5 | 97.5 | 0.5 | 2.9 | 50.7 | 49.1 | 46.8 |

(continued)

| Sample | Temp. [°C] | Elemental Fe [wt%] | NaOH/Fe molar ratio | L* | a* | b* | ΔE black | ΔE yellow | ΔE red |
|--------|------------|--------------------|---------------------|------|------|------|----------|-----------|--------|
| B | 300 | 0.1 | 5 | 96.2 | 1.4 | 4.0 | 49.6 | 47.4 | 45.3 |
| B | 500 | 0.1 | 5 | 94.7 | 1.2 | 3.9 | 48.0 | 47.0 | 44.0 |
| C | 125 | 0.5 | 1 | 89.3 | 4.3 | 16.1 | 45.8 | 33.4 | 40.4 |
| C | 300 | 0.5 | 1 | 86.2 | 5.5 | 16.8 | 43.4 | 31.7 | 37.5 |
| C | 500 | 0.5 | 1 | 85.4 | 5.7 | 16.6 | 42.6 | 31.6 | 36.7 |
| D | 125 | 0.5 | 5 | 88.6 | 4.6 | 17.2 | 45.7 | 32.1 | 40.1 |
| D | 300 | 0.5 | 5 | 85.2 | 5.9 | 17.1 | 42.7 | 31.1 | 36.7 |
| D | 500 | 0.5 | 5 | 84.4 | 5.8 | 16.8 | 41.8 | 31.2 | 35.9 |
| E | 125 | 1.0 | 1 | 85.1 | 8.0 | 30.9 | 50.3 | 17.6 | 43.7 |
| E | 300 | 1.0 | 1 | 75.9 | 11.3 | 29.5 | 43.4 | 18.0 | 35.8 |
| E | 500 | 1.0 | 1 | 75.5 | 11.4 | 29.3 | 43.0 | 18.3 | 35.4 |
| F | 125 | 1.0 | 5 | 83.6 | 7.3 | 21.3 | 43.4 | 26.5 | 36.9 |
| F | 300 | 1.0 | 5 | 80.5 | 8.5 | 21.6 | 41.2 | 25.7 | 34.3 |
| F | 500 | 1.0 | 5 | 79.3 | 9.0 | 21.8 | 40.5 | 25.5 | 33.4 |
| G | 125 | 3.0 | 1 | 76.0 | 11.7 | 33.1 | 46.2 | 14.5 | 38.7 |
| G | 300 | 3.0 | 1 | 68.3 | 14.2 | 31.8 | 41.4 | 19.1 | 33.4 |
| G | 500 | 3.0 | 1 | 67.4 | 14.2 | 31.2 | 40.5 | 20.1 | 32.4 |
| H | 125 | 3.0 | 5 | 75.1 | 12.9 | 34.4 | 46.9 | 13.6 | 39.1 |
| H | 300 | 3.0 | 5 | 68.2 | 19.4 | 35.1 | 45.9 | 18.5 | 36.3 |
| H | 500 | 3.0 | 5 | 67.7 | 19.7 | 34.9 | 45.7 | 19.0 | 36.0 |
| I | 125 | 5.0 | 1 | 72.0 | 15.4 | 41.5 | 51.5 | 10.2 | 43.6 |
| I | 300 | 5.0 | 1 | 60.8 | 18.0 | 36.2 | 43.4 | 22.5 | 35.0 |
| I | 500 | 5.0 | 1 | 59.8 | 18.0 | 35.7 | 42.6 | 23.6 | 34.3 |
| J | 125 | 5.0 | 5 | 69.1 | 14.5 | 36.8 | 46.0 | 14.9 | 38.2 |
| J | 300 | 5.0 | 5 | 69.3 | 16.8 | 38.9 | 48.5 | 14.2 | 40.1 |
| J | 500 | 5.0 | 5 | 68.8 | 16.7 | 38.3 | 47.8 | 14.8 | 39.4 |
| K | 125 | 7.5 | 1 | 66.4 | 18.5 | 44.3 | 52.4 | 15.2 | 44.3 |
| K | 300 | 7.5 | 1 | 54.4 | 20.1 | 36.5 | 42.9 | 28.5 | 34.7 |
| K | 500 | 7.5 | 1 | 53.4 | 19.9 | 34.8 | 41.2 | 29.9 | 33.0 |
| L | 125 | 7.5 | 5 | 56.9 | 10.4 | 29.4 | 33.4 | 28.4 | 28.3 |
| L | 300 | 7.5 | 5 | 52.3 | 29.5 | 40.1 | 50.6 | 33.5 | 40.3 |
| L | 500 | 7.5 | 5 | 51.8 | 30.2 | 40.2 | 51.0 | 34.2 | 40.6 |
| M | 125 | 10.0 | 1 | 61.9 | 19.0 | 40.6 | 47.9 | 20.2 | 39.6 |
| M | 300 | 10.0 | 1 | 54.1 | 19.7 | 35.8 | 42.1 | 28.9 | 34.0 |
| M | 500 | 10.0 | 1 | 52.0 | 19.5 | 33.6 | 39.8 | 31.5 | 31.8 |
| N | 125 | 10.0 | 5 | 49.2 | 5.4 | 19.2 | 20.8 | 41.2 | 20.9 |
| N | 300 | 10.0 | 5 | 52.5 | 27.6 | 41.6 | 50.8 | 32.0 | 41.1 |
| N | 500 | 10.0 | 5 | 51.0 | 27.4 | 39.3 | 48.6 | 33.6 | 38.9 |
| Z | 125 | 0.0 | -- | 76.7 | 10.4 | 32.1 | 45.6 | 15.3 | 38.5 |

(continued)

| Sample | Temp. [°C] | Elemental Fe [wt%] | NaOH/Fe molar ratio | L* | a* | b* | ΔE black | ΔE yellow | ΔE red |
|--------|-----------|--------------------|--------------------|------|------|------|---------|-----------|--------|
| Z | 500 | 0.0 | -- | 69.7 | 12.5 | 29.9 | 40.2 | 19.7 | 32.5 |

Table 6

| Sample | Temp. [°C] | Elemental Fe (wt%) | NaBH$_4$/Fe molar ratio | L* | a* | b* | ΔE black | ΔE yellow | ΔE red |
|--------|-----------|--------------------|-----------------------|------|------|------|---------|-----------|--------|
| BB | 125 | 0.1 | 5 | 98.0 | 0.4 | 2.4 | 51.2 | 49.7 | 47.3 |
| BB | 300 | 0.1 | 5 | 96.8 | 1.3 | 3.6 | 50.1 | 48.0 | 45.9 |
| BB | 500 | 0.1 | 5 | 95.7 | 1.1 | 3.2 | 49.0 | 48.0 | 44.9 |
| CC | 125 | 1.0 | 1 | 87.9 | 5.5 | 23.0 | 47.7 | 26.3 | 41.8 |
| CC | 300 | 1.0 | 1 | 81.8 | 8.0 | 23.4 | 43.2 | 24.1 | 36.5 |
| CC | 500 | 1.0 | 1 | 81.1 | 8.2 | 23.8 | 42.9 | 23.6 | 36.1 |
| DD | 125 | 1.0 | 5 | 82.5 | 1.1 | 8.6 | 36.9 | 39.9 | 33.5 |
| DD | 300 | 1.0 | 5 | 78.4 | 2.6 | 11.5 | 33.9 | 36.6 | 30.1 |
| DD | 500 | 1.0 | 5 | 82.5 | 5.6 | 17.4 | 40.4 | 30.4 | 34.6 |
| EE | 125 | 3.0 | 1 | 78.6 | 5.0 | 25.4 | 41.4 | 22.5 | 36.2 |
| EE | 300 | 3.0 | 1 | 69.0 | 8.9 | 27.0 | 36.6 | 22.6 | 30.4 |
| EE | 500 | 3.0 | 1 | 68.5 | 13.2 | 28.7 | 38.8 | 21.4 | 30.8 |
| FF | 125 | 3.0 | 5 | 56.5 | -0.5 | -2.2 | 9.7 | 55.4 | 17.8 |
| FF | 300 | 3.0 | 5 | 53.0 | -0.3 | -1.6 | 6.2 | 56.4 | 17.2 |
| FF | 500 | 3.0 | 5 | 65.6 | 19.1 | 21.7 | 34.8 | 30.0 | 23.3 |
| GG | 125 | 5.0 | 1 | 70.5 | 5.7 | 25.0 | 35.4 | 24.3 | 30.6 |
| GG | 300 | 5.0 | 1 | 61.0 | 8.2 | 24.0 | 29.7 | 29.5 | 24.7 |
| GG | 500 | 5.0 | 1 | 63.1 | 16.7 | 28.9 | 37.6 | 25.0 | 28.6 |
| HH | 125 | 5.0 | 5 | 55.0 | -0.9 | -1.4 | 8.2 | 55.4 | 17.8 |
| HH | 300 | 5.0 | 5 | 49.9 | -0.7 | -0.8 | 3.2 | 57.3 | 17.8 |
| HH | 500 | 5.0 | 5 | 54.1 | 7.5 | 9.8 | 14.7 | 45.0 | 11.9 |
| II | 125 | 10.0 | 1 | 68.4 | 14.1 | 36.9 | 45.6 | 15.2 | 38.0 |
| II | 300 | 10.0 | 1 | 60.7 | 16.6 | 34.5 | 41.3 | 23.1 | 33.3 |
| II | 500 | 10.0 | 1 | 58.0 | 17.1 | 32.2 | 38.7 | 26.6 | 30.5 |
| JJ | 125 | 10.0 | 5 | 39.9 | -0.4 | -1.9 | 7.1 | 63.7 | 22.0 |
| JJ | 300 | 10.0 | 5 | 37.7 | -0.2 | -0.5 | 9.2 | 64.0 | 23.1 |
| JJ | 500 | 10.0 | 5 | 54.5 | 17.9 | 26.0 | 33.0 | 33.2 | 24.1 |
| CC/10 eq. | 125 | 1.0 | 10 | 70.8 | -0.6 | -2.0 | 23.9 | 51.1 | 24.7 |
| CC/10 eq. | 300 | 1.0 | 10 | 67.6 | -0.2 | -1.1 | 20.7 | 50.8 | 22.1 |
| CC/10 eq. | 500 | 1.0 | 10 | 77.1 | 14.4 | 16.4 | 37.5 | 31.1 | 27.7 |
| FF/10 eq. | 125 | 3.0 | 10 | 55.4 | -1.0 | -4.3 | 9.3 | 57.8 | 18.7 |
| FF/10 eq. | 300 | 3.0 | 10 | 51.6 | -0.8 | -3.9 | 5.7 | 59.1 | 18.4 |
| FF/10 eq. | 500 | 3.0 | 10 | 63.0 | 18.4 | 18.4 | 30.9 | 33.9 | 19.0 |

Table 7

| Sample (comparative) | L* | a* | b* | ΔE black | ΔE yellow | ΔE red |
|---|---|---|---|---|---|---|
| 4.3 g SRCC + 0.7 g Ferroxide® Black 78P | 46.9 | 0.4 | -0.9 | 0.0 | 58.7 | 17.6 |
| 4.0 g SRCC + 1.0 g Pur Oxy Yellow BC | 79.0 | 10.6 | 47.2 | 58.7 | 0.0 | 52.2 |
| 4.3 g SRCC + 0.7 g Ferroxide® Red 226P | 53.5 | 16.5 | 2.0 | 17.6 | 52.2 | 0.0 |

A further big advantage of the compositions of the present invention is their efficiency as regards the amount of iron compound.

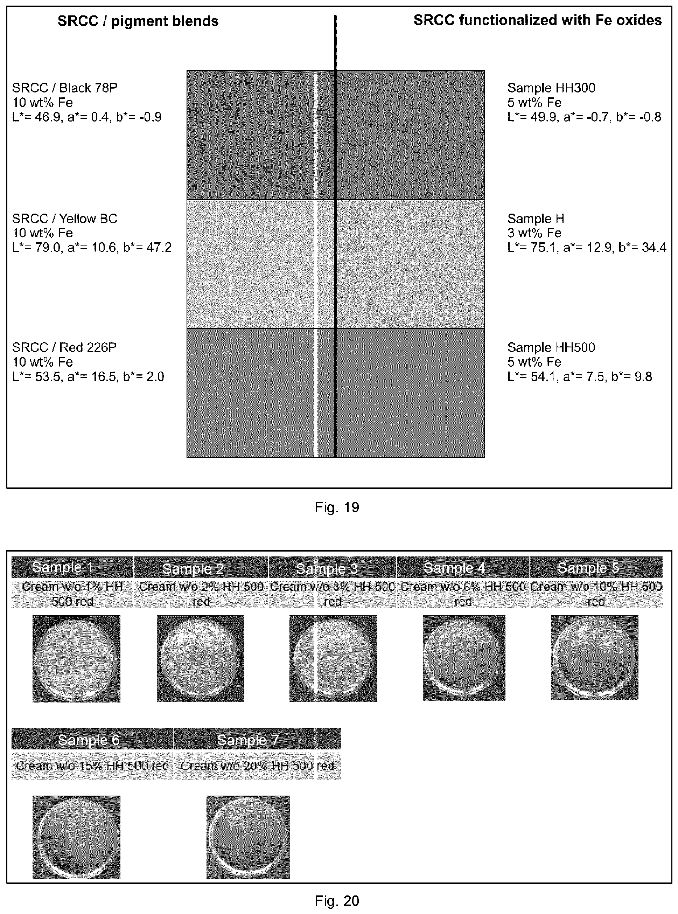

As can be taken from figure 19, in order to obtain a comparable colour, significantly less iron compound has to be used for the pigment according to the present invention compared with pigments consisting of surface-reacted calcium carbonate mixed with known pigments such as Ferroxide Black 78 P, Pur Oxy Yellow BC, or Ferroxide Red 226P.

Thus, e.g. a mixture of surface-reacted calcium carbonate and 10 wt% Pur Oxy Yellow BC leads to a comparable colour as an inventive sample at 3 wt% Fe, and a molar ratio of NaOH / Fe of 1 : 1 after drying at 125 °C. However, the inventive sample needs a third of the iron content.

A mixture of surface-reacted calcium carbonate and 10 wt% Ferroxide Black 78P leads to a comparable colour as an inventive sample at 5 wt% Fe, and a molar ratio of $NaBH_4$ / Fe of 5 : 1 calcined at a temperature of 300 °C. However, the inventive sample needs half of the iron content.

Finally, a mixture of surface-reacted calcium carbonate and 10 wt% Ferroxide Red 226P leads to a comparable colour as an inventive sample at 5 wt% Fe, and a molar ratio of $NaBH_4$ / Fe of 5 : 1 calcined at a temperature of 500 °C. However, the inventive sample needs half of the iron content.

### 4.3. UV/Vis/NIR reflectance characterization of the samples

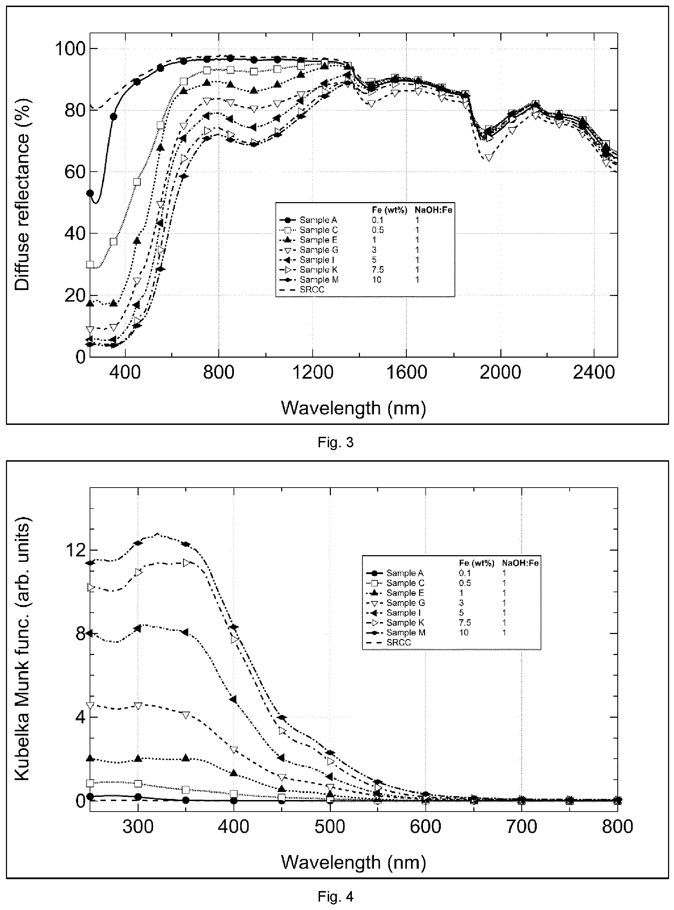

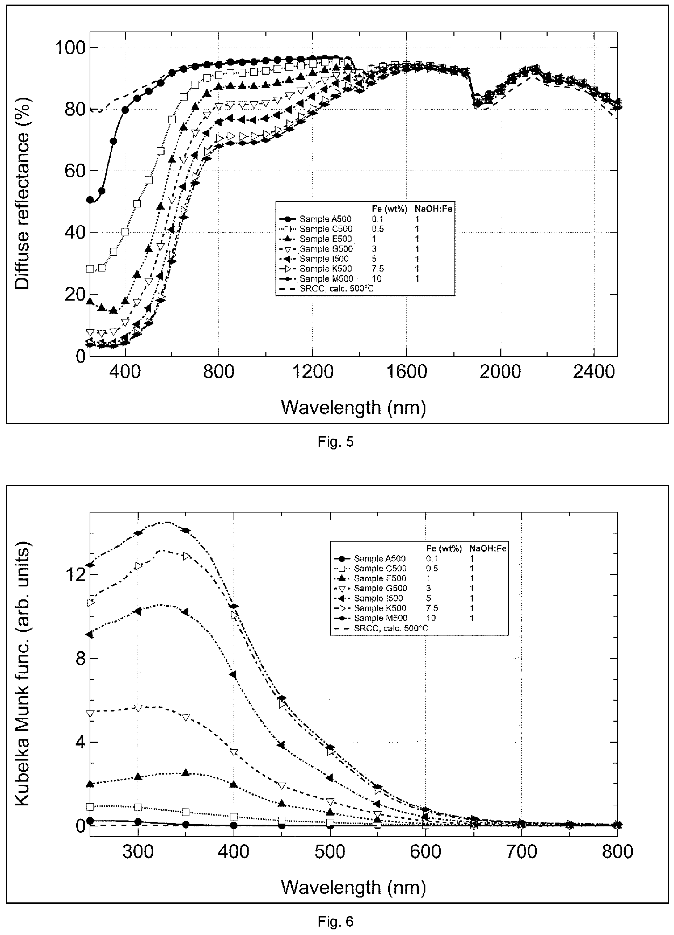

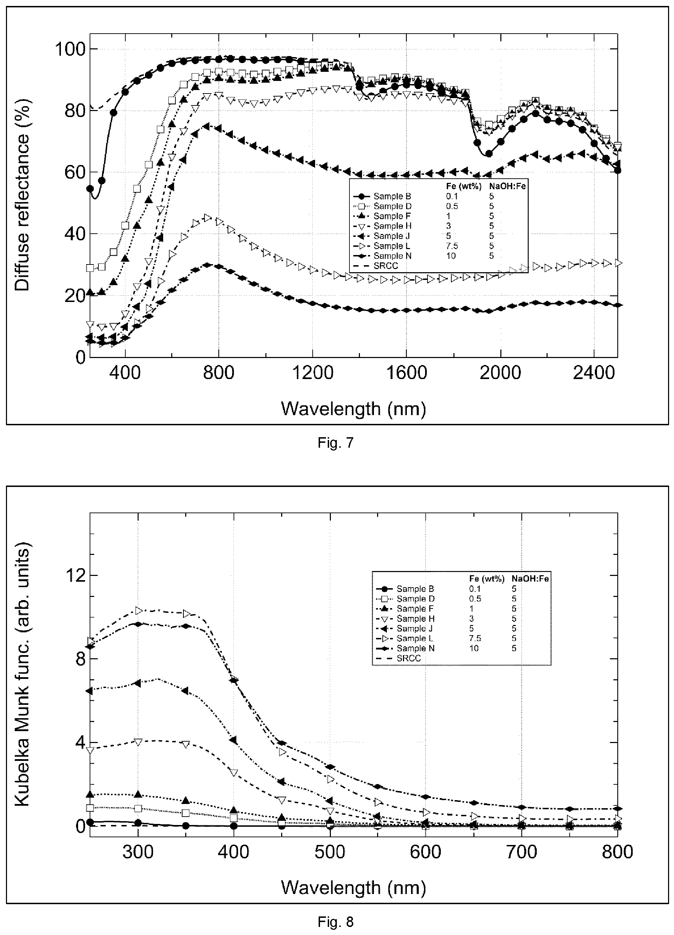

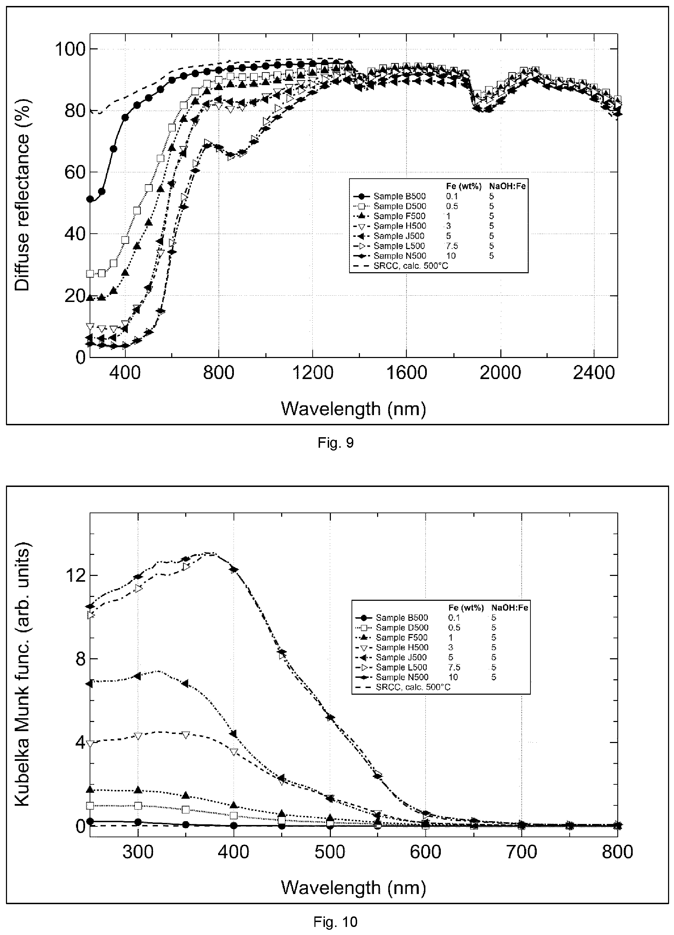

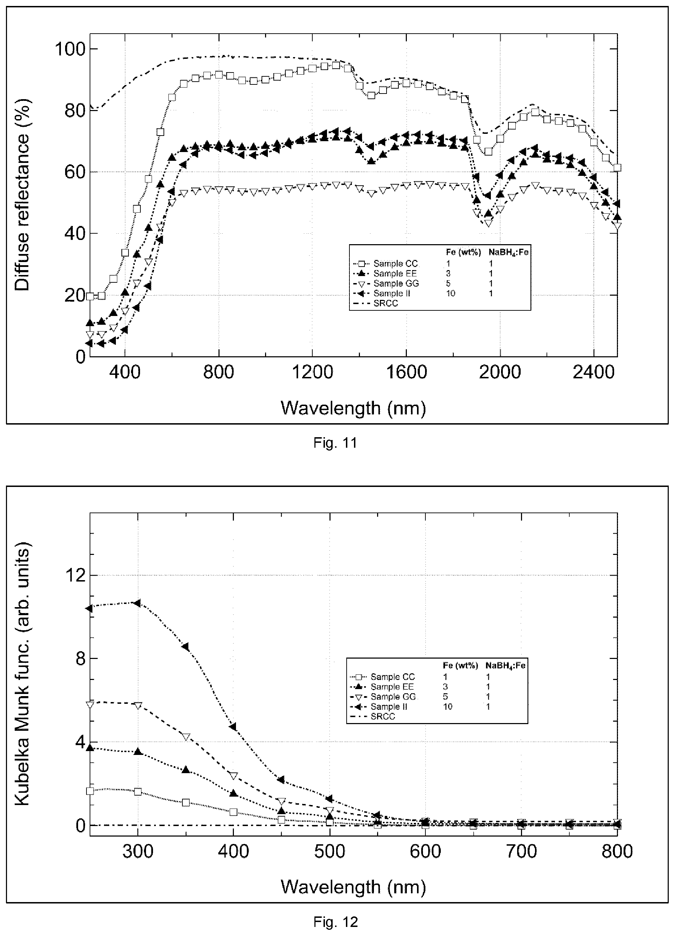

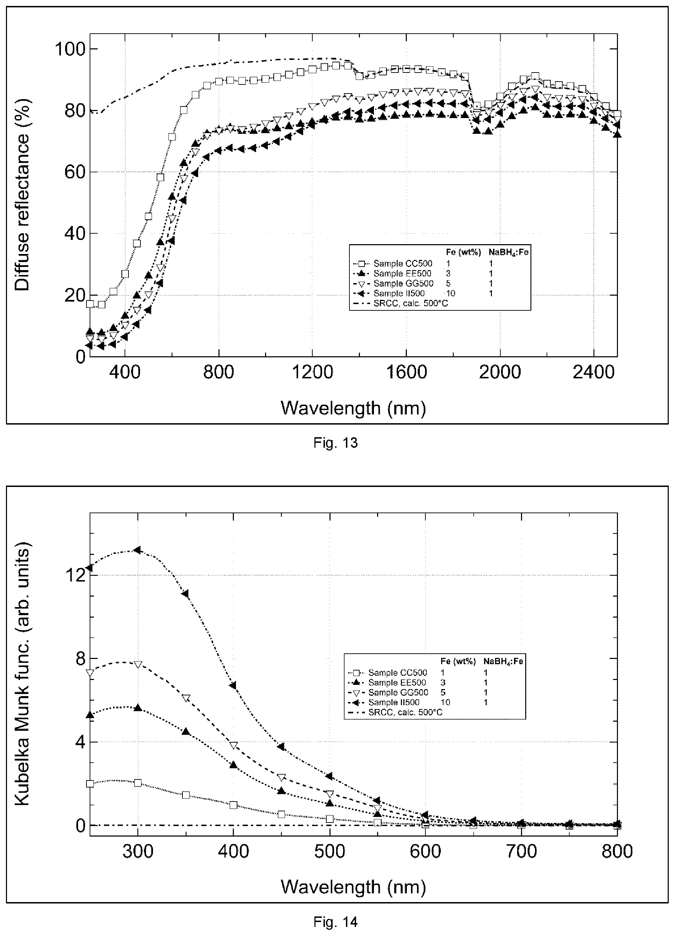

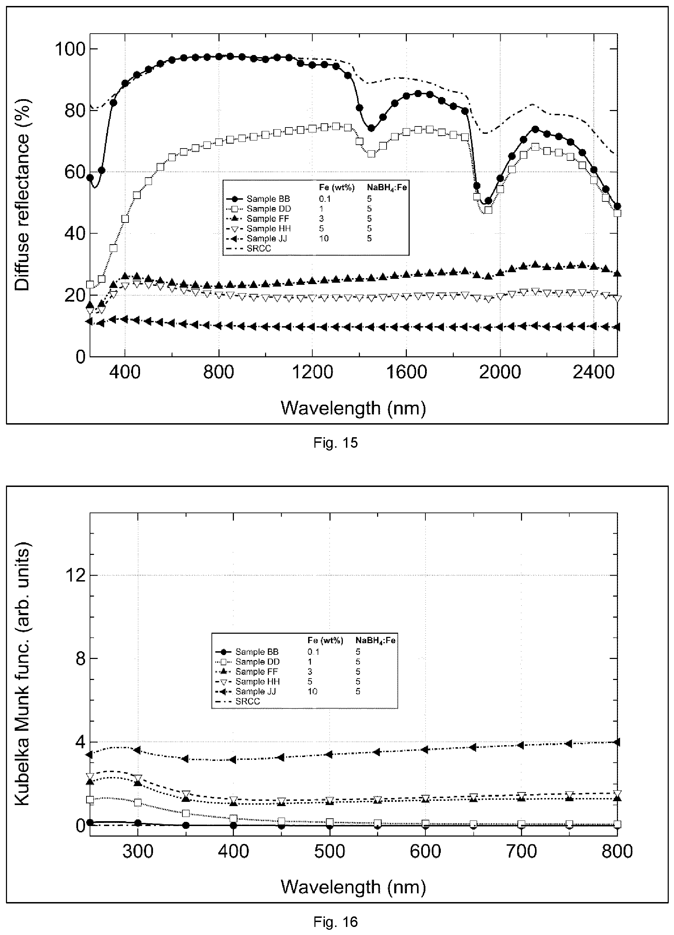

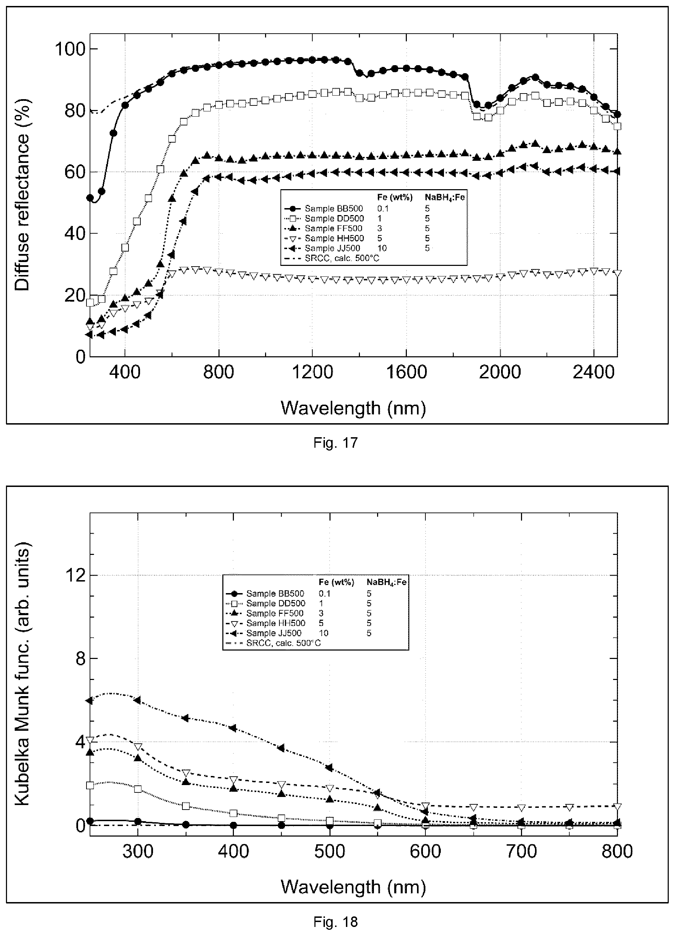

From figures 3 to 18, not only the effect of different iron species, the amount of iron species based on the dry weight of surface-reacted calcium carbonate, and the molar ratio of precipitation agent or reducing agents to iron in the UV, visible up to the NIR spectrum can be seen, but also the effect of calcination compared to the same samples before calcination, i.e. being only dried at 125 °C.

These figures clearly illustrate that for both, samples treated with precipitation agent (NaOH) and reducing agent ($NaBH_4$) an increase of the amount of iron species leads to a decrease of the diffuse reflectance corresponding to an increase of the absorption in the UV and visible range.

The diffuse reflectance decrease occurs also in the NIR range and is more evident at high treatment agent: Fe ratios, and especially for the samples treated with the reducing agent ($NaBH_4$). Upon calcination, the samples retain their UV absorption properties, which in some cases are even enhanced, while the modifications in the visible range correlate with the color change of the samples. In the NIR range, the spectra of the calcined samples resemble that of the SSRC. The inventive samples have therefore an improved UV protection compared to the SRCC. Additionally, the inventive samples have similar IR properties compared to the SRCC in all cases except for the samples treated with the reducing agent ($NaBH_4$) at high treatment agent : Fe ratios.

### 5. Cosmetic Formulations

In order to study the suitability of the inventive pigments in cosmetic applications, several formulations have been prepared and examined. The base formulations were prepared as follows:

### a) Water-in-Oil Cream (W/O Cream)

Table 8

| Ingredients | | Tradename / Supplier | % w/w |
|---|---|---|---|
| A | Water | | add. 100 |
| | Magnesium sulfate | | 1.0 |
| | Sodium chloride | Sigma Aldrich, Switzerland | 1.0 |
| | Glycerin | | 20.0 |

(continued)

| | Ingredients | | Tradename / Supplier | % w/w |
|---|---|---|---|---|
| B | Polyglyceryl-3 diisostearate | | Plurol Diisostearique CG (Gattefossé, France) | 5.0 |
| | Dicaprylyl carbonate | | Cetiol CC (BASF, Switzerland) | 10.5 |
| | Octyldodecyl myristate | | MOD (Gattefossé, France) | 4.5 |
| | Caprylic/capric triglycerides | | Labrafac CC MB (Gattefossé, France) | 1.0 |
| C | Fragrance (Parfum) | | Perfume (Hänseler AG, Switzerland) | q.s |
| | Leuconostoc Radish Root Ferment Filtrate (and) Aqua | | Leucidal (Hänseler AG, Switzerland) | 3.00 |

Phase A and B were heated separately at 80 °C. Subsequently, phase B was added to phase A while stirring. The mixture was cooled down at room temperature. Subsequently, phase C was added to the mixture and homgenized resulting in a mattifying cream.

**b) Oil-in-Water Cream (O/W Cream)**

Table 9

| | Ingredients | Tradename / Supplier | % w/w |
|---|---|---|---|
| A | Cetearyl alcohol | Lanette O (Cognis GmbH, Germany) | 2.00 |
| | Tribehenin PEG-20 esters | Emulium 22MB (Gattefossé, France) | 3.00 |
| | Prunus Amygdalus Dulcis (Almond) oil | Almond Oil (Hänseler AG, Switzerland) | 2.00 |
| | Macadamia Ternifolia seed oil | Macadamia Oil (Hänseler AG, Switzerland) | 3.00 |
| | Caprylic/capric triglyceride | Miglyol 812 (Hänseler AG, Switzerland) | 4.00 |
| | Octyldodecyl myristate | MOD (Gattefossé, France) | 4.00 |
| | Tocopheryl acetate | Copherol 1250C (Sigma Aldrich, Switzerland) | 1.00 |
| B | Aqua (water) | Water demineralized | add.100 |
| | Propanediol | Propanediol Zemea (Omya Inc. USA) | 5.00 |
| | Glycerin | Glycerin | 3.00 |
| | Xanthan gum | Xanthan Gum (Omya Hamburg GmbH, Germany) | 0.10 |
| | Sodium chloride | Sodium Chloride | 0.50 |
| | Allantoin | Allantoin EP (Omya Hamburg GmbH, Germany) | 0.10 |
| C | Fragrance (parfum) | Perfume (Omya AG, Switzerland) | q.s |
| | Leuconostoc Radish Root Ferment Filtrate (and) Aqua | Leucidal (Omya Inc. USA) | 3.00 |

Phase A and B were heated separately at 80 °C. Subsequently, phase B was added to phase A while stirring. The mixture was cooled down at room temperature. Subsequently, phase C was added to the mixture and homogenized resulting in a mattifying cream. The pH was adjusted to a pH of 6 using lactic acid (10%-solution), if necessary.

**c) Pigment addition**

To these creams, pigments according to the invention were added as mentioned in the below tables and investigated as regards their colors in W/O and O/W creams.

The composition and characteristics of the used pigments can be taken from the tables above, wherein, e.g. HH 500 means sample HH calcined at a temperature of 500 °C.

**5.1. Screening of red colored SRCC**

Table 10

| Sample | 1 | 2 | 3 | 4 | 5 | 6 | 7 |
|---|---|---|---|---|---|---|---|
| Description | Cream W/O x % HH 500 red | | | | | | |
| | 1 % | 2% | 3% | 6% | 10 % | 15 % | 20 % |
| Rx | 58,1 | 45,4 | 43,6 | 30,8 | 25,0 | 22,7 | 17,0 |
| Ry | 49,9 | 36,5 | 34,9 | 22,7 | 17,8 | 16,0 | 12,5 |
| Rz | 38,3 | 24,8 | 23,5 | 13,1 | 9,4 | 8,5 | 7,1 |
| L* | 76,0 | 66,9 | 65,6 | 54,8 | 49,2 | 46,9 | 42,0 |
| a* | 11,6 | 15,4 | 15,8 | 19,7 | 21,0 | 21,0 | 16,2 |
| b* | 13,3 | 17,2 | 17,3 | 20,6 | 21,4 | 20,7 | 17,1 |
| Delta E | 34,8 | 25,7 | 24,5 | 15,5 | 12,4 | 11,0 | 10,4 |

It can be seen from table 10 and Figure 20 that it is possible to finely tune the colour of a W/O cream in the red colour range.

**5.2. Screening of yellow colored SRCC**

Table 11

| Sample | 8 | 9 | 10 | 11 | 12 | 13 | 14 |
|---|---|---|---|---|---|---|---|
| Description | Cream W/O x % E300 yellow | | | | | | |
| | 1 % | 2% | 3% | 6% | 7% | 10 % | 15 % |
| Rx | 79,6 | 71,5 | 64,9 | 56,0 | 56,4 | 51,6 | 45,1 |
| Ry | 72,6 | 63,1 | 55,3 | 45,1 | 45,5 | 40,6 | 34,2 |
| Rz | 54,2 | 42,4 | 33,2 | 22,5 | 22,9 | 19,0 | 14,5 |
| L* | 88,2 | 83,5 | 79,2 | 72,9 | 73,2 | 69,9 | 65,1 |
| a* | 4,5 | 6,4 | 8,6 | 12,5 | 12,4 | 14,0 | 16,5 |
| b* | 16,7 | 21,3 | 25,7 | 31,7 | 31,4 | 33,1 | 34,9 |
| Delta E | 42,3 | 36,0 | 30,3 | 23,2 | 23,4 | 21,8 | 21,4 |

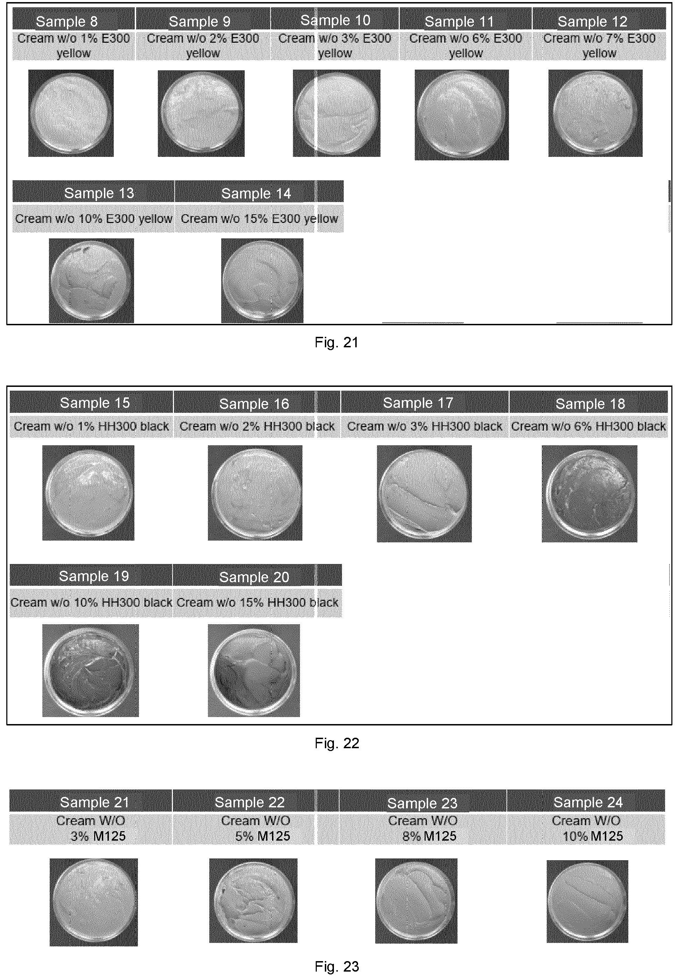

It can be seen from table 11 and Figure 21 that it is possible to finely tune the colour of a W/O cream in the yellow colour range.

**5.3. Screening of black colored SRCC**

Table 12

| Sample | 15 | 16 | 17 | 18 | 19 | 20 |
|---|---|---|---|---|---|---|
| Description | Cream W/O x % HH300 black | | | | | |
| | 1 % | 2% | 3% | 6% | 10 % | 15 % |
| Rx | 41,3 | 29,7 | 25,5 | 14,1 | 10,9 | 8,8 |

(continued)

| Sample | 15 | 16 | 17 | 18 | 19 | 20 |
|---|---|---|---|---|---|---|
| Description | Cream W/O x % HH300 black | | | | | |
|  | 1 % | 2% | 3% | 6% | 10 % | 15 % |
| Ry | 41,1 | 29,5 | 25,5 | 14,1 | 10,8 | 8,8 |
| Rz | 39,9 | 28,6 | 24,7 | 13,6 | 10,4 | 8,4 |
| L* | 70,2 | 61,3 | 57,5 | 44,4 | 39,3 | 35,5 |
| a* | -0,3 | -0,3 | -0,4 | -0,3 | -0,3 | -0,2 |
| b* | 1,5 | 1,4 | 1,2 | 1,2 | 1,2 | 1,1 |
| Delta E | 36,8 | 27,8 | 24,1 | 11,1 | 6,1 | 2,8 |

It can be seen from table 12 and Figure 22 that it is possible to finely tune the colour of a W/O cream in the grey colour range.

**5.4. Screening of brown colored SRCC**

**5.4.1. Water-in-oil Cream (W/O Cream)**

Table 13

| Sample | 21 | 22 | 23 | 24 |
|---|---|---|---|---|
| Description | Cream W/O x % M125 | | | |
|  | 3% | 5% | 8% | 10 % |
| Rx | 52,1 | 41,2 | 31,5 | 29,6 |
| Ry | 41,45 | 30,72 | 22,15 | 20,58 |
| Rz | 20,3 | 12,3 | 7,8 | 7,1 |
| L* | 70,5 | 62,3 | 54,2 | 52,5 |
| a* | 13,3 | 17,5 | 20,5 | 21,0 |
| b* | 31,7 | 35,5 | 35,5 | 35,3 |

It can be seen from table 13 and Figure 23 that it is possible to finely tune the colour of a W/O cream in the brown colour range.

**5.4.2. Oil-in-water Cream (O/W Cream)**

Table 14

| Sample | 25 | 26 | 27 | 28 |
|---|---|---|---|---|
| Description | Cream O/W x % M125 | | | |
|  | 3% | 5% | 8% | 10 % |
| Rx | 51,5 | 44,5 | 37,3 | 33,3 |
| Ry | 40,42 | 33,62 | 26,88 | 23,45 |
| Rz | 19,0 | 14,1 | 9,7 | 8,1 |
| L* | 69,8 | 64,7 | 58,9 | 55,5 |

(continued)

| Sample | 25 | 26 | 27 | 28 |
|---|---|---|---|---|
| Description | Cream O/W x % M125 | | | |
| | 3% | 5% | 8% | 10 % |
| a* | 14,5 | 16,9 | 19,6 | 20,7 |
| b* | 33,0 | 35,1 | 37,1 | 36,9 |

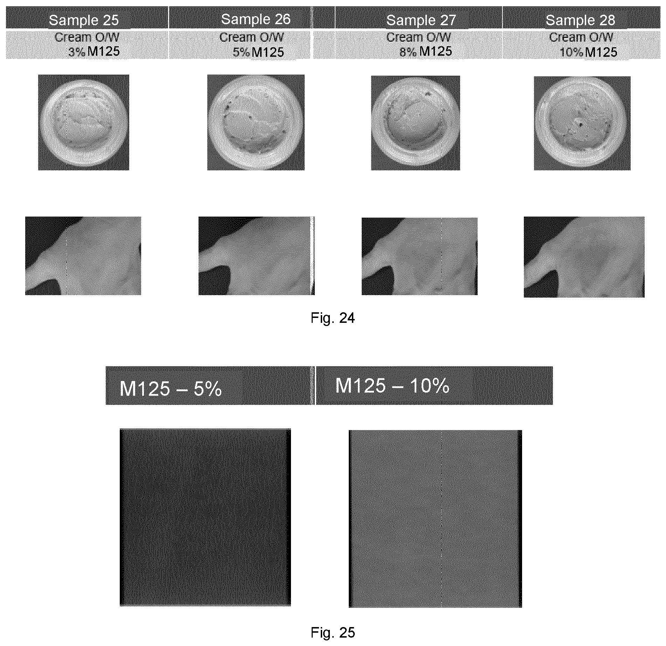

It can be seen from table 14 and Figure 24 that it is also possible to finely tune the colour of a O/W cream in the brown colour range, wherein the colours are nearly the same as in the corresponding W/O cream samples.

5.5. Coverage

In order to determine the covering power (coverage) of the pigment material, a base composition comprising different pigment concentrations of the pigment material, namely 5 and 10 wt%, were prepared. The covering power of the respective base compositions was determined by measuring the colour values ($Rx, Ry, Rz$) and then calculating the contrast ratio, as described above.

The base composition contains the ingredients listed in Table 15.

Table 15

| Ingredients | Tradenames (Suppliers) | Compound | Weight % (based on total weight of base colour) |
|---|---|---|---|
| | Demineralised water | Demineralised water | 40.0 |
| Dispersing agent | Calgon N new (BK Giulini) | Sodium polyphosphate | 0.2 |
| Thickener | Bermocoll EHM 200 (Akzo Nobel) | Cellulose ether | 1.0 |
| pH Regulation | Sodium hydroxide solution, 10 % (Sigma-Aldrich) | Sodium hydroxide solution | 0.6 |
| Defoamer | Byk 011 (Byk (Altana Group)) | Polymer | 2.0 |
| Film forming agent | Texanol (Eastman) | Isobutyric acid, ester with 2,2,4-trimethyl-1,3-pentanediol | 0.5 |
| Film forming agent | Butyldiglycol acetate (Sigma-Aldrich) | Ester | 0.5 |
| Film forming agent | Dowanol™ DPnB (Dow) | Dipropylene glycol n-butyl ether | 1.0 |
| Defoamer | Byk 019 (Byk (Altana Group)) | Polyether-modified polydimethylsiloxane | 0.5 |
| Rheology modifier | Coapur™ 2025 (Coatex (Arkema Group)) | Polyurethane based | 1.8 |
| Preservative | Mergal 723 K (Troy Chemical Company) | Benzoisothiazolinone | 0.2 |
| | Demineralized water | Demineralised water | 5.0 |
| Wetting and dispersing agent | Ecodis™ P 90 (Coatex (Arkema Group)) | Ammonium neutralized polyacrylate | 0.6 |
| Wetting agent | Disperbyk®-181 (Byk (Altana Group)) | Alkylolammonium salt of a polyfunctional polymer | 1.0 |

(continued)

| Ingredients | Tradenames (Suppliers) | Compound | Weight % (based on total weight of **base** colour) |
|---|---|---|---|
| Surfactant | Byk 349 (Byk (Altana Group)) | Polyether-modified siloxane | 0.4 |
| | Demineralized water | Demineralised water | 14.7 |
| Binding agent | Mowilith® DM 2425, 50 % (Celanese) | Aqueous copolymer dispersion based on vinyl acetate | 30.0 |
| Total | | | **100.0** |

The base composition was prepared as follows:

The demineralized water was added to a beaker, then, Calgon, Bermocoll and the sodium hydroxide solution were added under stirring with a lab dissolver until all ingredients were dissolved. Then the other ingredients listed in Table 16 up to Byk 349 were added while continuously stirring the mixture. Then the demineralized water was added and the resulting mixture was thoroughly mixed. Finally, the binding agent Mowilith was added during continuous stirring of the mixture at a speed of 100 rpm to obtain the final base colour.

This base composition was used for the preparation of formulations with different pigment concentrations.

As pigments, M125 and untreated SRCC were used in order to verify whether the treatment has an impact on the coverage.

The formulations were prepared by weighing the respective pigment material in the required amount and adding the respective amount of the base composition. Then the resulting mixtures were homogenized for 1 minute by use of a speed mixer at a speed of 3000 rpm. Then the mixture was mixed using a spatula and, subsequently, the mixture was again homogenized for 1 minute by use of a speed mixer at a speed of 3000 rpm. The resulting mixture was then used for the measurement of the colour values ($Rx$, $Ry$, $Rz$ ) which in turn were used for the calculation of the contrast ratio.

The contrast ratio (coverage) values for the used pigment materials at the different pigment concentrations are listed in Table 16.

Table 16

| wt% pigment based on the total weight of the formulation | Coverage (%) |
|---|---|
| 5 wt% M125 | 13 |
| 10 wt% M125 | 36 |
| 5 wt% SRCC | 13 |
| 10 wt% SRCC | 43 |

It can be seen from these results that the treatment of SRCC according to the invention does not essentially affect the coverage of the formulation. The coverage is furthermore illustrated in Figure 25.

**Claims**

1. A method for the manufacture of a pigment **characterized by** the steps of

a) providing at least one surface-reacted calcium carbonate,
b) providing at least one water-soluble iron compound,
c) providing at least one treatment agent,
d) combining the at least one surface-reacted calcium carbonate of step a) with the at least one water-soluble iron compound of step b) in an aqueous medium,
e) adding the at least one treatment agent to the mixture of step d),
f) dewatering the mixture of step e),
g) thermally treating the mixture of step f) at a temperature of from 80 to 150 °C,

wherein the surface-reacted calcium carbonate is a reaction product of ground natural calcium carbonate-containing mineral (GNCC) or precipitated calcium carbonate (PCC) with carbon dioxide and one or more $H_3O^+$ ion donors and wherein the carbon dioxide is formed in situ by the $H_3O^+$ ion donors treatment and/or is supplied from an external source, and

wherein the at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.05 to 40 wt%, based on the total dry weight of the surface-reacted calcium carbonate.

2. The method according to claim 1,
   **characterized in that** the at least one surface-reacted calcium carbonate of step a) has

   a) a volume median particle size $d_{50}$ (vol) in the range from of from 1 to 75 $\mu$m, preferably from 2 to 50 $\mu$m, more preferably 3 to 40 $\mu$m, even more preferably from 4 to 30 $\mu$m, and most preferably from 5 to 15 $\mu$m, and/or
   b) a top cut particle size $d_{98}$ (vol) of from 2 to 150 $\mu$m, preferably from 4 to 100 $\mu$m, more preferably 6 to 80 $\mu$m, even more preferably from 8 to 60 $\mu$m, and most preferably from 10 to 30 $\mu$m, and/or
   c) a specific surface area (BET) of from 10 $m^2/g$ to 200 $m^2/g$, preferably from 20 $m^2/g$ to 180 $m^2/g$, more preferably from 30 $m^2/g$ to 160 $m^2/g$, even more preferably from 45 $m^2/g$ to 150 $m^2/g$, most preferably from 50 $m^2/g$ to 100 $m^2/g$, measured by the BET nitrogen method, and/or
   d) an intra-particle intruded specific pore volume in the range from 0.1 to 2.3 $cm^3/g$, preferably from 0.2 to 2.0 $cm^3/g$, especially preferably from 0.4 to 1.8 $cm^3/g$ and most preferably from 0.6 to 1.6 $cm^3/g$, calculated from mercury porosimetry measurement.

3. The method according to any one of the preceding claims,
   **characterized in that** the at least one water-soluble iron compound of step b) is selected from the group comprising iron(II) sulfate; iron(III) sulfate; iron(II) halides, such as iron(II) chloride or iron(II) bromide; iron(III) halides, such as iron(III) chloride or iron(III) bromide; iron(II) nitrate; iron(III) nitrate; iron(II) phosphate; iron(III) phosphate; iron(II) oxalate; iron(III) oxalate; iron(II) acetate; iron(III) acetate; hydrates, and mixtures thereof.

4. The method according to any one of the preceding claims,
   **characterized in that** at least one water-soluble iron compound of step b) is added in an amount of from 0.05 to 40 wt%, preferably in an amount of from 0.1 to 30 wt%, more preferably in an amount of from 0.5 to 20 wt%, even more preferably in an amount of from 1 to 10 wt%, most preferably in an amount of from 3 to 7.5 wt%, especially preferably in an amount of from 5 wt% to 6 wt% relating to the iron content in relation to the total dry weight of the surface-reacted calcium carbonate.

5. The method according to any one of the preceding claims,
   **characterized in that** the at least one treatment agent of step c) is selected from precipitation agents forming a water-insoluble iron compound when combined with the water-soluble iron compound, preferably alkaline and alkaline earth hydroxides, such as sodium hydroxide, potassium hydroxide, calcium hydroxide; ammonia; and mixtures thereof.

6. The method according to any one of claims 1 to 4,
   **characterized in that** the at least one treatment agent of step c) is selected from reducing agents forming elemental iron when combined with the water-soluble iron compound, preferably sodium borohydride, lithium borohydride, sodium hydride, lithium aluminium hydride, hydrogen, hydrazine, sodium citrate; and mixtures thereof.

7. The method according to any one of the preceding claims,
   **characterized in that** at least one water-soluble iron compound of step b) is added in an amount such that the amount of water-insoluble iron compound and/or the amount of elemental iron resulting from the reaction of the at least one water-soluble iron compound of step b) and the at least one treatment agent of step c) is from 0.1 to 30 wt%, more preferably from 0.5 to 20 wt%, even more preferably from 1 to 10 wt%, most preferably from 3 to 7.5 wt%, especially preferably from 5 wt% to 6 wt% based on the total dry weight of the surface-reacted calcium carbonate.

8. The method according to any one of the preceding claims,
   **characterized in that** in relation to the iron content of the water-soluble iron compound of step b), the at least one treatment agent of step c) is provided in a molar ratio of treatment agent : Fe of from 1 : 1 to 15 : 1, preferably 1.5 : 1 to 10 : 1, more preferably 2 : 1 to 7 : 1, most preferably 3 : 1 to 5 : 1.

9. The method according to any one of the preceding claims,

**characterized in that** steps d) and/or e), independently from each other, are carried out under stirring and/or at a temperature of from 25 to 95 °C, preferably of from 30 to 75 °C, more preferably of from 40 to 65°C, most preferably at 50 °C.

10. The method according to any one of the preceding claims,
**characterized in that** dewatering step f) is carried out by filtration, centrifugation, spray drying, evaporation, optionally under vacuum.

11. The method according to any one of the preceding claims,
**characterized in that** thermal treatment step g) is carried out at a temperature of from 90 to 140 °C, preferably 100 to 130 °C, more preferably 110 to 125 °C.

12. The method according to any one of the preceding claims,
**characterized in that,** after thermal treatment step g), a further thermal treatment step h) is carried out at a temperature of from more than 150 to 600 °C, preferably of from 200 °C to 550 °C, more preferably of from 250 to 500 °C, most preferably of from 300 °C to 450 °C, especially preferably from 350 to 400 °C.

13. A method for the manufacture of a cosmetic product, paint or coating **characterized by** the steps of manufacturing a pigment according to steps a) to g) of claim 1, and

    i) adding the pigment obtained in step g) to a cosmetic formulation, paint or coating.

**Patentansprüche**

1. Verfahren zur Herstellung eines Pigments, **gekennzeichnet durch** die Schritte:

    a) Bereitstellen mindestens eines oberflächenreagierten Calciumcarbonats,
    b) Bereitstellen mindestens einer wasserlöslichen Eisenverbindung,
    c) Bereitstellen mindestens eines Behandlungsmittels,
    d) Vereinigen des mindestens einen oberflächenreagierten Calciumcarbonats aus Schritt a) mit der mindestens einen wasserlöslichen Eisenverbindung aus Schritt b) in einem wässrigen Medium,
    e) Zugeben des mindestens einen Behandlungsmittels zu der Mischung aus Schritt d),
    f) Entwässern der Mischung aus Schritt e),
    g) thermisches Behandeln der Mischung aus Schritt f) bei einer Temperatur von 80 bis 150 °C,

    wobei das oberflächenreagierte Calciumcarbonat ein Reaktionsprodukt eines gemahlenen natürlichen Calciumcarbonat-haltigen Minerals (GNCC) oder eines gefällten Calciumcarbonats (PCC) mit Kohlendioxid und einem oder mehreren $H_3O^+$-Ionendonatoren ist, und wobei das Kohlendioxid in situ durch Behandlung mit dem einen oder den mehreren $H_3O^+$-Ionendonatoren gebildet und/oder aus einer externen Quelle zugeführt wird, und
    wobei die mindestens eine wasserlösliche Eisenverbindung aus Schritt b) in einer solchen Menge zugegeben wird, dass die Menge der wasserunlöslichen Eisenverbindung und/oder die Menge an elementarem Eisen, die aus der Reaktion der mindestens einen wasserlöslichen Eisenverbindung aus Schritt b) mit dem mindestens einen Behandlungsmittel aus Schritt c) resultiert, 0,05 bis 40 Gew.-%, bezogen auf das Gesamttrockengewicht des oberflächenreagierten Calciumcarbonats, beträgt.

2. Verfahren nach Anspruch 1,
**dadurch gekennzeichnet, dass** das mindestens eine oberflächenreagierte Calciumcarbonat aus Schritt a) mindestens eines der folgenden Merkmale aufweist:

    a) eine volumenmittlere Partikelgröße $d_{50}$ (vol) im Bereich von 1 bis 75 $\mu$m, vorzugsweise von 2 bis 50 $\mu$m, noch bevorzugter von 3 bis 40 $\mu$m, noch bevorzugter von 4 bis 30 $\mu$m und am meisten bevorzugt von 5 bis 15 $\mu$m, und/oder
    b) eine obere Top Cut Partikelgröße $d_{98}$ (vol) im Bereich von 2 bis 150 $\mu$m, vorzugsweise von 4 bis 100 $\mu$m, bevorzugter von 6 bis 80 $\mu$m, noch bevorzugter von 8 bis 60 $\mu$m und am meisten bevorzugt von 10 bis 30 $\mu$m, und/oder
    c) eine spezifische Oberfläche (BET) im Bereich von 10 m²/g bis 200 m²/g, vorzugsweise von 20 m²/g bis 180

m$^2$/g, bevorzugter von 30 m$^2$/g bis 160 m$^2$/g, noch bevorzugter von 45 m$^2$/g bis 150 m$^2$/g, am meisten bevorzugt von 50 m$^2$/g bis 100 m$^2$/g, gemessen nach dem BET-Stickstoffverfahren, und/oder

d) ein intrapartikuläres introdiertes spezifisches Porenvolumen im Bereich von 0,1 bis 2,3 cm$^3$/g, vorzugsweise von 0,2 bis 2,0 cm$^3$/g, besonders bevorzugt von 0,4 bis 1,8 cm$^3$/g und am meisten bevorzugt von 0,6 bis 1,6 cm$^3$/g, berechnet aus einer Quecksilberporosimetrie-Messung.

3.  Verfahren nach einem der vorhergehenden Ansprüche,
    **dadurch gekennzeichnet, dass** die mindestens eine wasserlösliche Eisenverbindung aus Schritt b) ausgewählt ist aus der Gruppe umfassend Eisen(II)-sulfat, Eisen(III)-sulfat, Eisen(II)-halogeniden, wie Eisen(II)-chlorid oder Eisen(II)-bromid, Eisen(III)-halogeniden, wie Eisen(III)-chlorid oder Eisen(III)-bromid, Eisen(II)-nitrat, Eisen(III)-nitrat, Eisen(II)-phosphat, Eisen(III)-phosphat, Eisen(II)-oxalat, Eisen(III)-oxalat, Eisen(II)-acetat, Eisen(III)-acetat, Hydraten und Mischungen davon.

4.  Verfahren nach einem der vorhergehenden Ansprüche,
    **dadurch gekennzeichnet, dass** die mindestens eine wasserlösliche Eisenverbindung aus Schritt b) in einer Menge von 0,05 bis 40 Gew.-%, vorzugsweise in einer Menge von 0,1 bis 30 Gew.-%, bevorzugter in einer Menge von 0,5 bis 20 Gew.-%, noch bevorzugter in einer Menge von 1 bis 10 Gew.-%, am meisten bevorzugt von 3 bis 7,5 Gew.-%, besonders bevorzugt in einer Menge von 5 bis 6 Gew.-%, bezogen auf den Eisengehalt in Bezug auf das Gesamttrockengewicht des oberflächenreagierten Calciumcarbonats, zugegeben wird.

5.  Verfahren nach einem der vorhergehenden Ansprüche,
    **dadurch gekennzeichnet, dass** das mindestens eine Behandlungsmittel aus Schritt c) ausgewählt ist aus Fällungsmitteln, die beim Vereinigen mit der wasserlöslichen Eisenverbindung eine wasserunlösliche Eisenverbindung bilden, vorzugsweise Alkalihydroxide und Erdalkalihydroxide, wie Natriumhydroxid, Kaliumhydroxid oder Calciumhydroxid, Ammoniak und Mischungen davon.

6.  Verfahren nach einem der Ansprüche 1 bis 4,
    **dadurch gekennzeichnet, dass** das mindestens eine Behandlungsmittel aus Schritt c) ausgewählt ist aus Reduktionsmitteln, die beim Vereinigen mit der wasserlöslichen Eisenverbindung elementares Eisen bilden, vorzugsweise Natriumborhydrid, Lithiumborhydrid, Natriumhydrid, Lithiumaluminiumhydrid, Wasserstoff, Hydrazin, Natriumcitrat sowie Mischungen davon.

7.  Verfahren nach einem der vorhergehenden Ansprüche,
    **dadurch gekennzeichnet, dass** die mindestens eine wasserlösliche Eisenverbindung aus Schritt b) in einer solchen Menge zugegeben wird, dass die Menge der wasserunlöslichen Eisenverbindung und/oder die Menge an elementarem Eisen, die aus der Reaktion der mindestens einen wasserlöslichen Eisenverbindung aus Schritt b) mit dem mindestens einen Behandlungsmittel aus Schritt c) resultiert, von 0,1 bis 30 Gew.-%, bevorzugter von 0,5 bis 20 Gew.-%, noch bevorzugter von 1 bis 10 Gew.-%, am meisten bevorzugt von 3 bis 7,5 Gew.-%, besonders bevorzugt von 5 bis 6 Gew.-%, bezogen auf das Gesamttrockengewicht des oberflächenreagierten Calciumcarbonats, beträgt.

8.  Verfahren nach einem der vorhergehenden Ansprüche,
    **dadurch gekennzeichnet, dass** das mindestens eine Behandlungsmittel aus Schritt c), bezogen auf den Eisengehalt der wasserlöslichen Eisenverbindung aus Schritt b), in einem molaren Verhältnis von Behandlungsmittel : Fe von 1 : 1 bis 15 : 1, vorzugsweise von 1,5 : 1 bis 10 : 1, bevorzugter von 2 : 1 bis 7 : 1, am meisten bevorzugt von 3 : 1 bis 5 : 1, bereitgestellt wird.

9.  Verfahren nach einem der vorhergehenden Ansprüche,
    **dadurch gekennzeichnet, dass** die Schritte d) und/oder e) unabhängig voneinander, unter Rühren und/oder bei einer Temperatur von 25 bis 95 °C, vorzugsweise von 30 bis 75 °C, bevorzugter von 40 bis 65 °C, am meisten bevorzugt bei 50 °C durchgeführt werden.

10. Verfahren nach einem der vorhergehenden Ansprüche,
    **dadurch gekennzeichnet, dass** der Entwässerungsschritt f) durch Filtration, Zentrifugation, Sprühtrocknung oder Verdampfung, optional unter Vakuum, durchgeführt wird.

11. Das Verfahren nach einem der vorhergehenden Ansprüche,
    **dadurch gekennzeichnet, dass** der thermische Behandlungsschritt g) bei einer Temperatur von 90 bis 140 °C, vorzugsweise von 100 bis 130 °C, bevorzugter von 110 bis 125 °C, durchgeführt wird.

**12.** Das Verfahren nach einem der vorhergehenden Ansprüche, **dadurch gekennzeichnet, dass** nach dem thermischen Behandlungsschritt g) ein weiterer thermischer Behandlungsschritt h) bei einer Temperatur von mehr als 150 bis 600 °C, vorzugsweise von 200 °C bis 550 °C, bevorzugter von 250 bis 500 °C, am meisten bevorzugt von 300 °C bis 450 °C, besonders bevorzugt von 350 bis 400 °C durchgeführt wird.

**13.** Ein Verfahren zur Herstellung eines kosmetischen Produkts, einer Farbe oder einer Beschichtung, **gekennzeichnet durch** die Schritte:
Herstellen eines Pigments gemäß den Schritten a) bis g) nach Anspruch 1, und

i) Zugeben des in Schritt g) erhaltenen Pigments zu einer kosmetischen Formulierung, einer Farbe oder einer Beschichtung.

## Revendications

**1.** Procédé de fabrication d'un pigment **caractérisé par** les étapes consistant à :

a) fournir au moins un carbonate de calcium traité par réaction en surface,
b) fournir au moins un composé de fer hydrosoluble,
c) fournir au moins un agent de traitement,
d) combiner l'au moins un carbonate de calcium traité par réaction en surface de l'étape a) avec le composé de fer hydrosoluble de l'étape b) en milieu aqueux,
e) ajouter l'au moins un agent de traitement au mélange de l'étape d),
f) déshydrater le mélange de l'étape e),
g) traiter thermiquement le mélange de l'étape f) à une température de 80 à 150 °C,

dans lequel le carbonate de calcium traité par réaction en surface est un produit de réaction d'un minéral naturel broyé contenant du carbonate de calcium (GNCC) ou de carbonate de calcium précipité (PCC) avec du dioxyde de carbone et un ou plusieurs donneurs d'ions $H_3O^+$ et dans lequel le dioxyde de carbone est formé in situ par le traitement avec des donneurs d'ions $H_3O^+$ et/ou provient d'une source externe, et dans lequel l'au moins un composé de fer hydrosoluble de l'étape b) est ajouté en une quantité telle que la quantité de composé de fer hydrosoluble et/ou la quantité de fer élémentaire résultant de la réaction de l'au moins un composé de fer hydrosoluble de l'étape b) et de l'au moins un agent de traitement de l'étape c) est de 0,05 à 40 % en poids, en se basant sur le poids sec total du carbonate de calcium traité par réaction en surface.

**2.** Procédé selon la revendication 1, **caractérisé en ce que** l'au moins un carbonate de calcium traité par réaction en surface de l'étape a) a

a) une taille de particule médiane en volume $d_{50}$ (vol) comprise dans la plage allant de 1 à 75 μm, de préférence de 2 à 50 μm, plus préférablement de 3 à 40 μm, encore plus préférablement de 4 à 30 μm, et de manière préférée entre toutes de 5 à 15 μm, et/ou
b) une taille de particule de coupe supérieure ($d_{98}$) (vol) de 2 à 150 μm, de préférence de 4 à 100 μm, plus préférablement de 6 à 80 μm, encore plus préférablement de 8 à 60 μm, et de manière préférée entre toutes de 10 à 30 μm, et/ou
c) une surface spécifique (BET) de 10 m²/g à 200 m²/g, de préférence de 20 m²/g à 180 m²/g, plus préférablement de 30 m²/g à 160 m²/g, encore plus préférablement de 45 m²/g à 150 m²/g, de manière préférée entre toutes de 50 m²/g à 100 m²/g, mesurée par la méthode BET à l'azote, et/ou
d) un volume poreux spécifique intrudé intraparticulaire compris dans la plage allant de 0,1 à 2,3 cm³/g, de préférence de 0,2 à 2,0 cm³/g, de préférence en particulier de 0,4 à 1,8 cm³/g et de manière préférée entre toutes de 0,6 à 1,6 cm³/g, calculé par mesure de porosimétrie au mercure.

**3.** Procédé selon l'une quelconque des revendications précédentes, **caractérisé en ce que** l'au moins un composé de fer hydrosoluble de l'étape b) est choisi dans le groupe comprenant le sulfate de fer(II); le sulfate de fer(III); les halogénures de fer(II), tels que le chlorure de fer(II) ou le bromure de fer(II); les halogénures de fer(III), tels que le chlorure de fer(III) ou le bromure de fer(III); le nitrate de fer(II); le nitrate de fer(III); le phosphate de fer(II); le phosphate de fer(III); l'oxalate de fer(II); l'oxalate de fer(III); l'acétate de fer(II); l'acétate de fer(III); les hydrates, et des mélanges de ceux-ci.

4. Procédé selon l'une quelconque des revendications précédentes, **caractérisé en ce que** l'au moins un composé de fer hydrosoluble de l'étape b) est ajouté en une quantité de 0,05 à 40 % en poids, de préférence en une quantité de 0,1 à 30 % en poids, plus préférablement en une quantité de 0,5 à 20 % en poids, encore plus préférablement en une quantité de 1 à 10 % en poids, de manière préférée entre toutes en une quantité de 3 à 7,5 % en poids, de préférence en particulier en une quantité de 5 % en poids à 6 % en poids rapportée à la teneur en fer par rapport au poids sec total du carbonate de calcium traité par réaction en surface.

5. Procédé selon l'une quelconque des revendications précédentes, **caractérisé en ce que** l'au moins un agent de traitement de l'étape c) est choisi parmi les agents de précipitation formant un composé de fer insoluble dans l'eau lorsqu'il est combiné avec le composé de fer hydrosoluble, de préférence les hydroxydes alcalins et alcalino-terreux, tels que l'hydroxyde de sodium, l'hydroxyde de potassium, l'hydroxyde de calcium; l'ammoniac; et des mélanges de ceux-ci.

6. Procédé selon l'une quelconque des revendications 1 à 4,
**caractérisé en ce que** l'au moins un agent de traitement de l'étape c) est choisi parmi les agents réducteurs formant du fer élémentaire lorsqu'ils sont combinés avec le composé de fer hydrosoluble, de préférence le borohydrure de sodium, le borohydrure de lithium, l'hydrure de sodium, l'hydrure de lithium et d'aluminium, l'hydrogène, l'hydrazine, le citrate de sodium; et des mélanges de ceux-ci.

7. Procédé selon l'une quelconque des revendications précédentes, **caractérisé en ce que** l'au moins un composé de fer hydrosoluble de l'étape b) est ajouté en une quantité telle que la quantité de composé de fer insoluble dans l'eau et/ou la quantité de fer élémentaire résultant de la réaction de l'au moins un composé de fer hydrosoluble de l'étape b) et de l'au moins un agent de traitement de l'étape c) est de 0,1 à 30 % en poids, plus préférablement de 0,5 à 20 % en poids, encore plus préférablement de 1 à 10 % en poids, de manière préférée entre toutes de 3 à 7,5 % en poids, de préférence en particulier de 5 % en poids à 6 % en poids en se basant sur le poids sec total du carbonate de calcium traité par réaction en surface.

8. Procédé selon l'une quelconque des revendications précédentes, **caractérisé en ce que** par rapport à la teneur en fer du composé de fer hydrosoluble de l'étape b), l'au moins un agent de traitement de l'étape c) est fourni selon un rapport molaire agent de traitement:Fe de 1:1 à 15:1, de préférence de 1,5:1 à 10:1, plus préférablement de 2:1 à 7:1, de manière préférée entre toutes de 3:1 et 5:1.

9. Procédé selon l'une quelconque des revendications précédentes,
**caractérisé en ce que** les étapes d) et/ou e), indépendamment l'une de l'autre, sont réalisées sous agitation et/ou à une température de 25 à 95 °C, de préférence de 30 à 75 °C, plus préférablement de 40 à 65 °C, de manière préférée entre toutes à 50 °C.

10. Procédé selon l'une quelconque des revendications précédentes,
**caractérisé en ce que** l'étape de déshydratation f) est réalisée par filtration, centrifugation, séchage par atomisation, évaporation, éventuellement sous vide.

11. Procédé selon l'une quelconque des revendications précédentes,
**caractérisé en ce que** l'étape de traitement thermique g) est réalisée à une température de 90 à 140 °C, de préférence de 100 à 130 °C, plus préférablement de 110 à 125 °C.

12. Procédé selon l'une quelconque des revendications précédentes,
**caractérisé en ce que**, après l'étape de traitement thermique g), une étape de traitement thermique h) supplémentaire est réalisée à une température de plus de 150 °C à 600 °C, de préférence de 200 °C à 550 °C, plus préférablement de 250 °C à 500 °C, de manière préférée entre toutes de 300 °C à 450 °C, de préférence en particulier de 350 °C à 400 °C.

13. Procédé de fabrication d'un produit cosmétique, d'une peinture ou d'un revêtement **caractérisé par** les étapes consistant à
fabriquer un pigment selon les étapes a) à g) de la revendication 1, et

 i) ajouter le pigment obtenu à l'étape g) à une formulation cosmétique, une peinture ou un revêtement.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

| SRCC / pigment blends | | SRCC functionalized with Fe oxides |
|---|---|---|
| SRCC / Black 78P<br>10 wt% Fe<br>L*= 46.9, a*= 0.4, b*= -0.9 | | Sample HH300<br>5 wt% Fe<br>L*= 49.9, a*= -0.7, b*= -0.8 |
| SRCC / Yellow BC<br>10 wt% Fe<br>L*= 79.0, a*= 10.6, b*= 47.2 | | Sample H<br>3 wt% Fe<br>L*= 75.1, a*= 12.9, b*= 34.4 |
| SRCC / Red 226P<br>10 wt% Fe<br>L*= 53.5, a*= 16.5, b*= 2.0 | | Sample HH500<br>5 wt% Fe<br>L*= 54.1, a*= 7.5, b*= 9.8 |

Fig. 19

| Sample 1 | Sample 2 | Sample 3 | Sample 4 | Sample 5 |
|---|---|---|---|---|
| Cream w/o 1% HH 500 red | Cream w/o 2% HH 500 red | Cream w/o 3% HH 500 red | Cream w/o 6% HH 500 red | Cream w/o 10% HH 500 red |

| Sample 6 | Sample 7 |
|---|---|
| Cream w/o 15% HH 500 red | Cream w/o 20% HH 500 red |

Fig. 20

| Sample 8 | Sample 9 | Sample 10 | Sample 11 | Sample 12 |
|---|---|---|---|---|
| Cream w/o 1% E300 yellow | Cream w/o 2% E300 yellow | Cream w/o 3% E300 yellow | Cream w/o 6% E300 yellow | Cream w/o 7% E300 yellow |

| Sample 13 | Sample 14 | |
|---|---|---|
| Cream w/o 10% E300 yellow | Cream w/o 15% E300 yellow | |

Fig. 21

| Sample 15 | Sample 16 | Sample 17 | Sample 18 |
|---|---|---|---|
| Cream w/o 1% HH300 black | Cream w/o 2% HH300 black | Cream w/o 3% HH300 black | Cream w/o 6% HH300 black |

| Sample 19 | Sample 20 | |
|---|---|---|
| Cream w/o 10% HH300 black | Cream w/o 15% HH300 black | |

Fig. 22

| Sample 21 | Sample 22 | Sample 23 | Sample 24 |
|---|---|---|---|
| Cream W/O 3% M125 | Cream W/O 5% M125 | Cream W/O 8% M125 | Cream W/O 10% M125 |

Fig. 23

Fig. 24

Fig. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3192838 A1
- WO 2018019692 A1
- EP 3360601 A1
- WO 0039222 A1
- WO 2004083316 A1
- WO 2005121257 A2
- WO 2009074492 A1
- EP 2264108 A1
- EP 2264109 A1
- US 20040020410 A1
- WO 2004083316 A

**Non-patent literature cited in the description**

- **GANE, P.A.C.** ; **KETTLE, J.P.** ; **MATTHEWS, G.P.** ; **RIDGWAY, C.J.** Void Space Structure of Compressible Polymer Spheres and Consolidated Calcium Carbonate Paper-Coating Formulations. *Industrial and Engineering Chemistry Research*, 1996, vol. 35 (5), 1753-1764
- *CHEMICAL ABSTRACTS*, 7782-63-0
- *CHEMICAL ABSTRACTS*, 1310-73-2
- *CHEMICAL ABSTRACTS*, 16940-66-2
- *CHEMICAL ABSTRACTS*, 1317-61-9
- *CHEMICAL ABSTRACTS*, 51274-00-1
- *CHEMICAL ABSTRACTS*, 1309-37-1